# EUROPEAN PATENT APPLICATION

(11) **EP 4 549 910 A1**
(43) Date of publication of application: **07.05.2025**
(21) Application number: 24210114.5
(22) Date of filing: 31.10.2024
(51) Int. Cl.: G01N 15/10, G01N 15/14, G01N 15/1433, G01N 21/64, G01N 33/493

(54) **PARTICLE DETECTION METHOD, PARTICLE DETECTION APPARATUS, AND PROGRAM**

(30) Priority: 06.11.2023 JP 2023189678
(71) Applicant: SYSMEX CORPORATION, Kobe-shi Hyogo 651-0073 (JP)
(72) Inventor: MASAGO, Akinori, Kobe-shi, Hyogo, 651-0073 (JP); TSUBOI, Kazuya, Nagoya-shi, Aichi, 453-0801 (JP)
(74) Representative: Hoffmann Eitle

(57) **Abstract**

Disclosed is a particle detection method comprising: irradiating a urine sample containing multiple types of particles with excitation light to cause autofluorescence from mulberry bodies; dispersing fluorescence generated from the urine sample irradiated with the excitation light into fluorescence in a first wavelength band and fluorescence in a second wavelength band, and acquiring fluorescence images for each of the particles, the first wavelength band and the second wavelength band being included in a wavelength band of the autofluorescence; and extracting a particle whose reference information based on the fluorescence in the first wavelength band and the fluorescence in the second wavelength band obtained from the corresponding fluorescence image is included in a range corresponding to the mulberry bodies.

## Description

### CROSS REFERENCE TO RELATED APPLICATIONS

This application claims priority from prior Japanese Patent Application No. 2023-189678, filed on November 6, 2023, entitled "PARTICLE DETECTION METHOD, PARTICLE DETECTION APPARATUS, AND PROGRAM", the entire content of which is incorporated herein by reference.

The present invention relates to a particle detection method, a particle detection apparatus, and a program.

Fabry disease is one type of genetic disease and is developed through accumulation of a lipid called globotriaosylceramide (Gb3) in lysosomes which are places for digestion within cells. When Fabry disease is developed, the lysosomes malfunction so that various organs experience disorders. Although the causal relationship between development of Fabry disease and urinary mulberry bodies has been conventionally known, since the amount of urinary mulberry bodies contained in a urinary sediment is very small, it is difficult to detect the mulberry bodies through a urinary sediment test.

Further, although urinary mulberry bodies have been visually checked by using a microscope, since the forms of urinary mulberry bodies are similar to the forms of red blood cells and fungi, it is highly difficult to distinguish these forms through this method. Therefore, examiners are required to have advanced skills to achieve this distinguishment.

Tomoko Namba, "Enabling Early Diagnosis of Rare Disease through Urine Test", [online], January 8, 2021, Resou, [searched on October 14, 2022], the Internet <URL: https://resou.osaka-u.ac.jp/ja/research/2021/20210108_4> describes that staining of a urinary sediment has made it clear that urinary mulberry bodies are derived from lysosomes which are present in the cytoplasm of a podocyte and in which Gb3 has been accumulated.

Gb3 is expressed with respect to not only mulberry bodies but also tubular cells. Therefore, in the distinguishing method based on the detection of the stained Gb3, it is difficult to accurately distinguish mulberry bodies and tubular cells from each other.

In view of these problems, an object of the present invention is to provide a particle detection method, a particle detection apparatus, and a program that enable mulberry bodies to be more accurately distinguished from other particles.

### SUMMARY OF THE INVENTION

A particle detection method of the present invention comprises: irradiating a urine sample containing multiple types of particles with excitation light to cause autofluorescence from mulberry bodies (S2); dispersing fluorescence generated from the urine sample irradiated with the excitation light into fluorescence in a first wavelength band and fluorescence in a second wavelength band, and acquiring fluorescence images for each of the particles, the first wavelength band and the second wavelength band being included in a wavelength band of the autofluorescence (S3); and extracting a particle whose reference information based on the fluorescence in the first wavelength band and the fluorescence in the second wavelength band obtained from the corresponding fluorescence image is included in a range corresponding to the mulberry bodies (S4).

With the particle detection method of the present invention, the particle whose reference information based on the fluorescence in the first and second wavelength bands is included in the range corresponding to mulberry bodies is extracted, whereby mulberry bodies can be more accurately distinguished from other particles.

A particle detection apparatus (1) of the present invention comprises: a light source (121) for irradiating a urine sample containing multiple types of particles with excitation light to cause autofluorescence from mulberry bodies; an imaging unit (20) for dispersing fluorescence generated from the urine sample irradiated with the excitation light into fluorescence in a first wavelength band and fluorescence in a second wavelength band, and for acquiring fluorescence images for each of the particles, the first wavelength band and the second wavelength band being included in a wavelength band of the autofluorescence; and a processor (11) for extracting a particle whose reference information based on the fluorescence in the first wavelength band and the fluorescence in the second wavelength band obtained from the corresponding fluorescence image is included in a range corresponding to the mulberry bodies.

With the particle detection apparatus of the present invention, the particle whose reference information based on the fluorescence in the first and second wavelength bands is included in the range corresponding to mulberry bodies is extracted, whereby mulberry bodies can be more accurately distinguished from other particles.

A program (12a) of the present invention is configured to cause a computer to execute a process of detecting particles in a urine sample containing multiple types of particles, the process comprising: extracting a particle whose reference information based on fluorescence in a first wavelength band and fluorescence in a second wavelength band obtained from corresponding fluorescence image is included in a range corresponding to mulberry bodies, wherein the first wavelength band and the second wavelength band being included in a wavelength band of autofluorescence, the autofluorescence is caused from a urine sample to which excitation light to cause the autofluorescence from the mulberry bodies is irradiated.

With the program of the present invention, the particle whose reference information based on the fluorescence in the first and second wavelength bands is included in the range corresponding to mulberry bodies is extracted, whereby mulberry bodies can be more accurately distinguished from other particles.

According to the present invention, mulberry bodies can be more accurately distinguished from other particles.

### BRIEF DESCRIPTION OF THE DRAWINGS

FIG. 1 is a block diagram illustrating a functional configuration of a particle detection apparatus according to an embodiment.
FIG. 2 is a diagram illustrating a schematic configuration of an imaging unit according to the embodiment.
FIG. 3 is a flowchart illustrating a process related to a particle detection method by a processor according to the embodiment.
FIG. 4 is a diagram showing a scattergram generated in a first extraction step according to the embodiment.
FIG. 5 is a diagram showing a bright-field image of a particle included in a range to be extracted in the scattergram of the first extraction step according to the embodiment.
FIG. 6 is a diagram showing a bright-field image of a particle included in a range other than the range to be extracted in the scattergram of the first extraction step according to the embodiment.
FIG. 7 is a diagram showing a scattergram generated in a second extraction step according to the embodiment.
FIG. 8 is a diagram showing a bright-field image of a particle included in a range other than a range to be extracted in the scattergram of the second extraction step according to the embodiment.
FIG. 9 is a diagram showing a bright-field image of a particle included in a range other than the range to be extracted in the scattergram of the second extraction step according to the embodiment.
FIG. 10 is a diagram showing a bright-field image of a particle included in the range to be extracted in the scattergram of the second extraction step according to the embodiment.
FIG. 11 is a diagram showing a scattergram generated in a third extraction step according to the embodiment.
FIG. 12 is a diagram showing a bright-field image of a particle included in a range other than a range to be extracted in the scattergram of the third extraction step according to the embodiment.
FIG. 13 is a diagram showing a bright-field image of a particle included in the range to be extracted in the scattergram of the third extraction step according to the embodiment.
FIG. 14 shows a bright field image of a cell-shaped mulberry body, according to the embodiment.
FIG. 15 is a diagram illustrating discrimination of the cell-shaped mulberry bodies contained in a urine sample according to the embodiment.
FIG. 16 is a table showing a number of particles, a number of mulberry bodies, and a proportion of the mulberry bodies contained in each range in the first to third extraction steps according to the embodiment.
FIG. 17 shows scattergrams obtained in the first to third extraction steps for the urine sample shown in FIG. 16 according to the embodiment.
FIG. 18 shows scattergrams obtained in the first to third extraction steps for the urine sample shown in FIG. 16 according to the embodiment.
FIG. 19 shows scattergrams obtained in the first to third extraction steps for the urine sample shown in FIG. 16 according to the embodiment.
FIG. 20 shows scattergrams obtained in the first to third extraction steps for the urine sample shown in FIG. 16 according to the embodiment.
FIG. 21 shows scattergrams obtained in the first to third extraction steps for the urine sample shown in FIG. 16 according to the embodiment.
FIG. 22 shows scattergrams obtained in the first to third extraction steps for the urine sample shown in FIG. 16 according to the embodiment.
FIG. 23 is a diagram showing a schematic configuration of a screen for displaying a particle image according to the embodiment.
FIG. 24 is a diagram showing a schematic configuration of a screen for displaying particle images according to the embodiment.
FIG. 25 illustrates a bright-field image of a particle determined by an operator to be a mulberry body, according to the embodiment.
FIG. 26 is a diagram showing a schematic configuration of a screen in a state where the operator has finished selecting a mulberry body according to the embodiment.
FIG. 27 is a diagram showing a schematic configuration of a screen displaying a history and changes in count results of mulberry bodies for the same subject according to the embodiment.
FIG. 28 is a diagram illustrating a schematic configuration of a screen for displaying particle images according to a modified example of the embodiment.
FIG. 29 is a diagram showing a first extraction step in which an average luminance is used according to a first modification.
FIG. 30 is a diagram showing a third extraction step in which a maximum luminance is used according to a second modification.
FIG. 31 is a diagram showing first and third extraction steps in which a total luminance is used according to a third modification.
FIG. 32 is a diagram showing a second extraction step in which only bright field contrast is used according to a fourth modification.
FIG. 33 is a diagram showing a third extraction step in which only average luminance is used according to a fifth modification.
FIG. 34 is a diagram showing a third extraction step in which only maximum luminance is used according to a sixth modification.
FIG. 35 is a diagram showing first and second extraction steps according to a seventh modified example.
FIG. 36 is a diagram showing a third extraction step according to the seventh modification and a modification of the seventh modification.
FIG. 37 is a flowchart showing a process related to a particle detection method by a processor according to an eighth modification.
FIG. 38 is a flowchart showing a process related to a particle detection method by a processor according to a ninth modification.

### DETAILED DESCRIPTION OF THE PREFERRED EMBODIMENTS

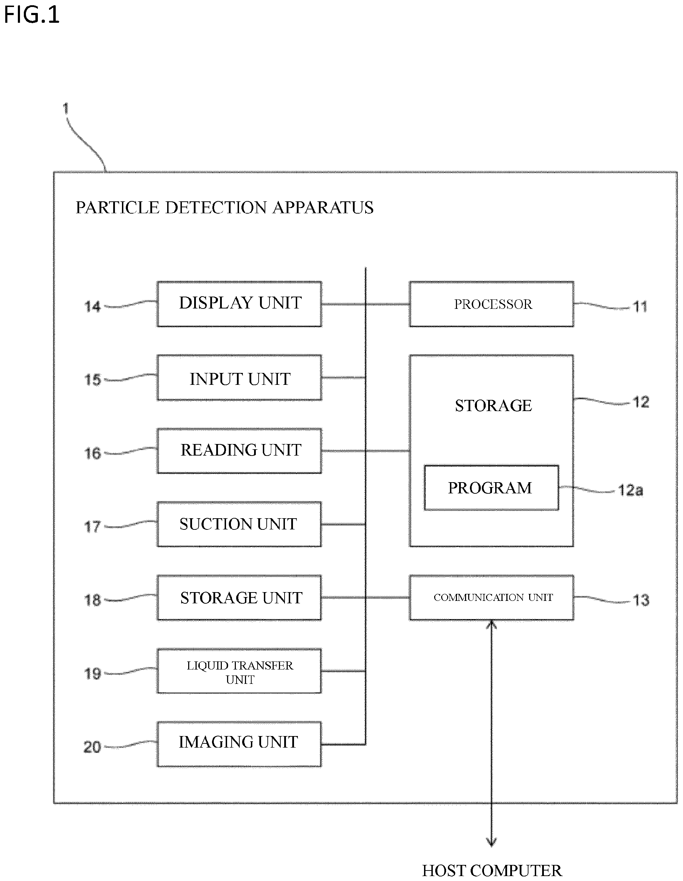

FIG. 1 is a block diagram showing a functional configuration of a particle detection apparatus 1 according to an embodiment of the present invention.

The particle detection apparatus 1 is an apparatus that detects particles contained in a urine specimen collected from a subject and distinguishes mulberry bodies contained in the urine specimen from particles other than mulberry bodies contained in the urine specimen. The particle detection apparatus 1 includes a processor 11, a storage 12, a communication unit 13, a display unit 14, an input unit 15, a reading unit 16, a suction unit 17, a storage unit 18, a liquid transfer unit 19, and an imaging unit 20.

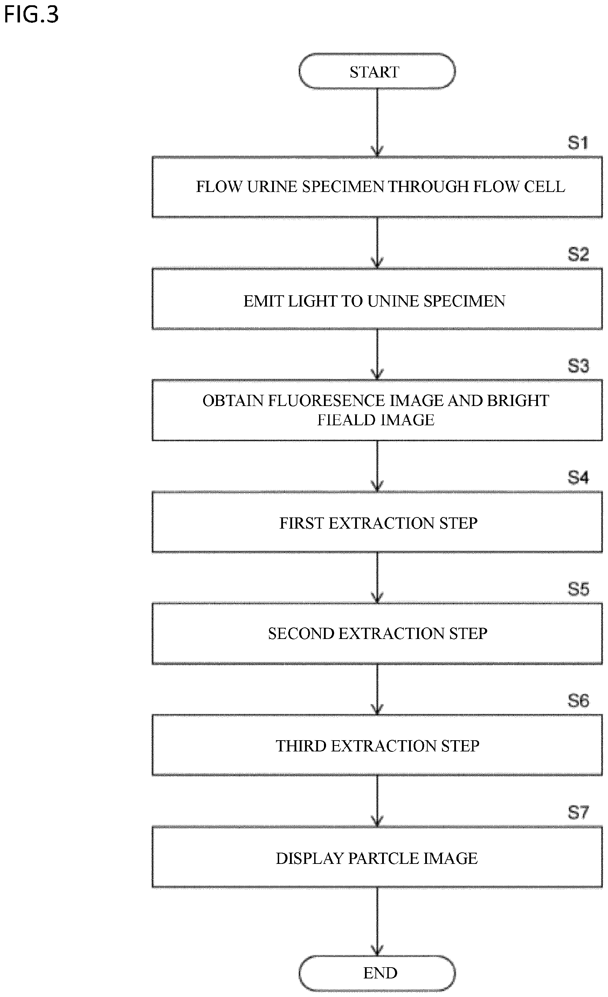

The processor 11 comprises, for example, an FPGA or a CPU. The processor 11 receives a signal outputted from each unit of the particle detection apparatus 1 and controls each unit of the particle detection apparatus 1. The storage 12 comprises, for example, an HDD or an SSD. The storage 12 stores therein a program 12a which causes the processor 11 to execute a process described later with reference to FIG. 3. The processor 11 reads the program 12a stored in the storage 12 and executes the process shown in FIG. 3 according to the program 12a. The program 12a may be stored in the storage 12 via a storing medium or may be stored in the storage 12 by being received from another computer via the communication unit 13.

The communication unit 13 is, for example, a communication interface based on the Ethernet standard. Via the communication unit 13, the processor 11 receives subject information corresponding to a specimen ID or the like from a host computer and transmits a counting result of particles or the like to the host computer.

The display unit 14 comprises, for example, a liquid crystal display. The input unit 15 comprises, for example, a keyboard, a pointing device including a mouse or a touch panel, etc. An operator operates the input unit 15 to operate operation sections such as a button in a screen displayed on the display unit 14. The display unit 14 and the input unit 15 may be integrated with each other. For example, the particle detection apparatus 1 may include a touch-panel type display as the display unit 14 and the input unit 15.

The reading unit 16 comprises, for example, a barcode reader. The reading unit 16 obtains a specimen ID by reading a barcode from a barcode label provided on a container containing a urine specimen. The container from which the specimen ID has been read is set in the particle detection apparatus 1.

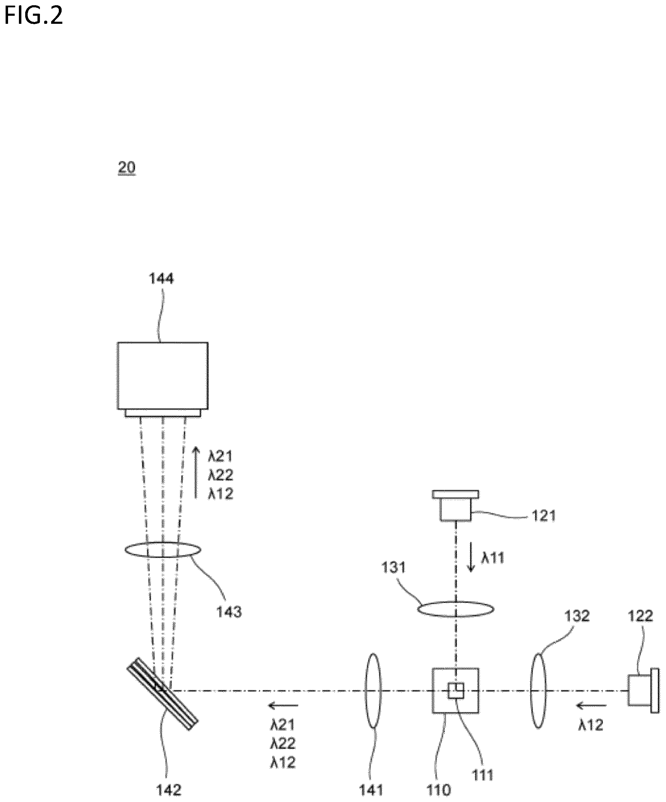

The suction unit 17 suctions the urine specimen through a nozzle from the container having been set in the particle detection apparatus 1. The storage unit 18 stores the urine specimen suctioned by the suction unit 17. The liquid transfer unit 19 transfers the urine specimen stored in the storage unit 18 via a tube and flows through a flow path 111 (see FIG. 2) of the flow cell 110. The imaging unit 20 images the urine specimen flowing through the flow path 111.

FIG. 2 schematically shows a configuration of the imaging unit 20.

The imaging unit 20 includes a flow cell 110, light sources 121,122, condenser lenses 131,132, a condenser lens 141, an optical unit 142, a condenser lens 143, and a camera 144.

The light sources 121,122 emit lights to the urine specimen flowing through the flow path 111 of the flow cell 110. The light sources 121,122 comprise, for example, a semiconductor laser light source and emits a laser light.

The light emitted from the light source 121 is excitation light having a wavelength λ11 and configured to generate autofluorescence from mulberry bodies in the urine specimen. The wavelength λ11 indicates a wavelength at which an intensity of the light emitted from the light source 121 is maximized. The light having the wavelength λ11 and emitted from the light source 121 includes not only light having the wavelength at which the intensity of the light becomes maximum but also light having wavelength near the wavelength at which the intensity of the light becomes maximum. That is, the light having the wavelength λ11 and emitted from the light source 121 is light in a wavelength band including the wavelength λ11. Light in a wavelength band including the wavelength λ may be referred to as light having the wavelength λ in this specification. In general, when mulberry bodies are irradiated with excitation light having a wavelength of from 350 nm or more and 550 nm or less, autofluorescence is generated from the mulberry bodies. Therefore, the wavelength λ11 is set to be 350 nm or more and 550 nm or less. In the present embodiment, the wavelength λ11 is 405 nm.

The light emitted from the light source 122 is light having a wavelength λ12 for obtaining bright field images of the particles in the urine specimen. The wavelength λ12 indicates a wavelength at which an intensity of the light emitted from the light source 122 becomes maximum. The light having the wavelength λ12 and emitted from the light source 122 includes not only light having the wavelength at which the intensity of the light becomes maximum but also light having wavelength near the wavelength at which the intensity of the light becomes maximum. The light having the wavelength λ12 is light in a wavelength band that is suitable for obtaining a bright field image and that is wider than the wavelength band of the light having the wavelength λ11.

The condenser lenses 131,132 condense the lights emitted from the light sources 121,122, respectively. The urine specimen flowing through the flow path 111 of the flow cell 110 is irradiated with the light having the wavelength λ11 and emitted from the light source 121 and the light having the wavelength λ12 and emitted from the light source 122.

When the urine specimen flowing through the flow cell 110 is irradiated with the light having the wavelength λ11, fluorescence is generated from the particles in the urine specimen. When the urine specimen flowing through the flow cell 110 is irradiated with the light having the wavelength λ12, this light is partially blocked by the particles in the urine specimen and partially transmitted therethrough.

When mulberry bodies are contained in the urine specimen, the fluorescence generated from the particles in the urine specimen includes autofluorescence in a predetermined wavelength band generated from the mulberry bodies. When the wavelength λ11 of the excitation light is 405 nm as described above, the wavelength band of the autofluorescence generated from the mulberry bodies is 435 nm or more and 560 nm or less. Therefore, the imaging unit 20 of the present embodiment is configured to image fluorescence in wavelength band including wavelength λ21 that is 435 nm or more and 505 nm or less (fluorescence having the wavelength λ21) and fluorescence in wavelength band including wavelength λ22 that is 505 nm or more and 560 nm or less (fluorescence having the wavelength λ22) so that the autofluorescence having the wavelength of 435 nm or more and 560 nm or less generated from the mulberry bodies can be imaged. The wavelengths λ21 and λ21 are wavelengths at which the intensity of the light is maximized in the corresponding wavelength band, respectively.

The condenser lens 141 condenses: the fluorescences having the wavelengths λ21 and λ22 and generated from the urine specimen flowing through the flow path 111 of the flow cell 110; and the light having the wavelength λ12 and transmitted through the urine specimen flowing through the flow path 111 of the flow cell 110. The optical unit 142 has a configuration in which three dichroic mirrors have been combined. The three dichroic mirrors of the optical unit 142 reflect the fluorescence having the wavelength λ21, the fluorescence having the wavelength λ22, and the light having the wavelength λ12 at angles slightly different from one another so that the fluorescences and the light are apart from one another on a light reception surface of the camera 144. The condenser lens 143 condenses the fluorescence having the wavelength λ21, the fluorescence having the wavelength λ22, and the light having the wavelength λ12, respectively.

The camera 144 is, for example, a TDI (Time Delay Integration) camera. The camera 144 images the fluorescence having the wavelength λ21, the fluorescence having the wavelength λ22, and the light having the wavelength λ12 and outputs, as imaging signals, a first fluorescence image and a second fluorescence image respectively corresponding to the fluorescences having the wavelengths λ21 and λ22 and a bright field image corresponding to the light having the wavelength λ12. The processor 11 in FIG. 1 distinguishes mulberry bodies contained in the urine specimen from other particles by using the first fluorescence images, the second fluorescence images, and the bright field images taken by the camera 144.

FIG. 3 is a flowchart showing a process regarding a particle detection method to be performed by the processor 11.

The processor 11 executes the program 12a stored in the storage 12, to perform the process shown in FIG. 3.

In step S1, the processor 11 controls the liquid transfer unit 19 to cause a urine specimen sucked from a container and stored in the storage unit 18 to flow through the flow path 111 of the flow cell 110. In the present embodiment, there is no need for a process of staining particles in a urine specimen flowing through the flow path 111 of the flow cell 110. In step S2, the processor 11 controls the light sources 121,122 to emit light to the urine specimen flowing through the flow path 111. Consequently, the urine specimen is irradiated with the light (excitation light) having the wavelength λ11, and the fluorescence having the wavelength λ21 and the fluorescence having the wavelength λ22 are generated from the particles in the urine specimen. In addition, the urine specimen is irradiated with the light having the wavelength λ12, and the light having the wavelength λ12 is transmitted through the particles in the urine specimen. The fluorescence having the wavelength λ21 and generated from the urine specimen, the fluorescence having the wavelength λ22 and generated from the urine specimen, and the light having the wavelength λ12 and transmitted through the urine specimen are dispersed by the optical unit 142, and the three apart lights are irradiated onto the light reception surface of the camera 144 in a separated state.

In step S3, the processor 11 controls the camera 144 to image each of a plurality of particles in the urine specimen, i.e., to image the fluorescence having the wavelength λ21 and generated from the urine specimen, the fluorescence having the wavelength λ22 and generated from the urine specimen, and the light having the wavelength λ12 and transmitted through the urine specimen. Consequently, the processor 11 obtains, for each of the particles in the urine specimen, a first fluorescence image, a second fluorescence image, and a bright field image. The processing unit 11 stores the obtained first fluorescence images, second fluorescence images, and bright field images in the storage 12.

Here, when the particle shown in the bright field image obtained by the camera 144 is smaller than a predetermined size, the processor 11 does not have to store the bright field image, the first fluorescence image, and the second fluorescence image corresponding to the particle in the storage 12. Specifically, when a number of pixels identified as constituting a region of a particle in the bright field image is equal to or smaller than the threshold value, the processor 11 does not have to store the bright field image and the first fluorescence image and the second fluorescence image corresponding to the bright field image in the storage 12. The threshold value is set to a number of pixels corresponding to a size that is smaller than a size of a typical mulberry body. Consequently, a number of particles to be subjected to the first extraction step is decreased, whereby processing load applied to the first to third extraction steps on the subsequent stage can be decreased. The above threshold value may be appropriately set according to, for example, the processing load applied to the first to third extraction steps, the lower limit of a bright field size of a range R2 set in the second extraction step, and the like.

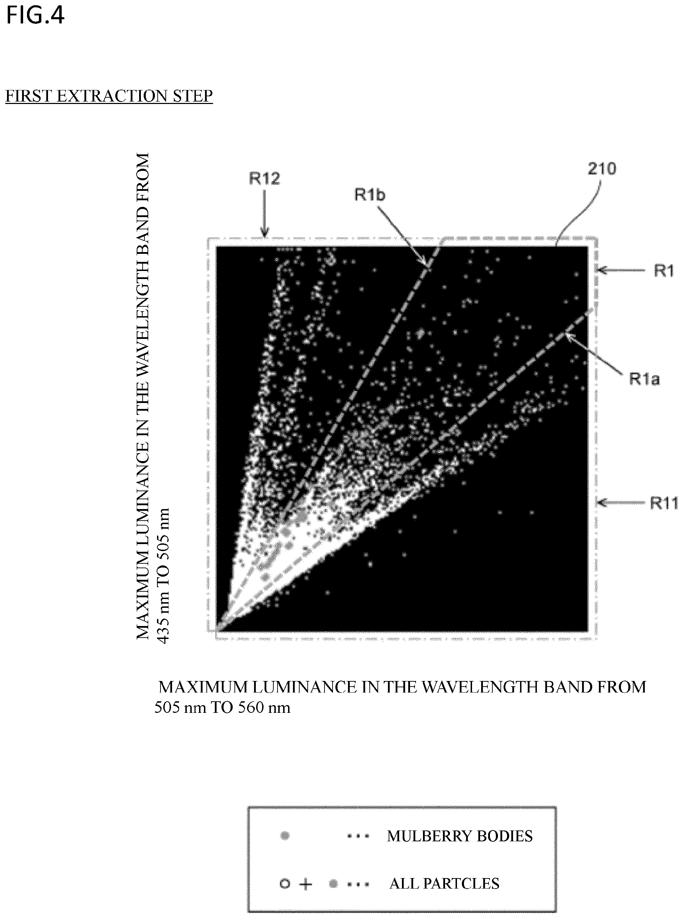

In step S4, the processor 11 performs the first extraction step regarding mulberry bodies on the basis of the first and second fluorescence images of all the particles obtained in step S3. In step S5, the processor 11 performs the second extraction step regarding mulberry bodies on the basis of the bright field images of particles extracted through the first extraction step in step S4. In step S6, the processor 11 performs the third extraction step regarding mulberry bodies on the basis of the first fluorescence images and the bright field images of particles extracted through the second extraction step in step S5. The first to third extraction steps will be described later in detail with reference to FIG. 4 to FIG. 13.

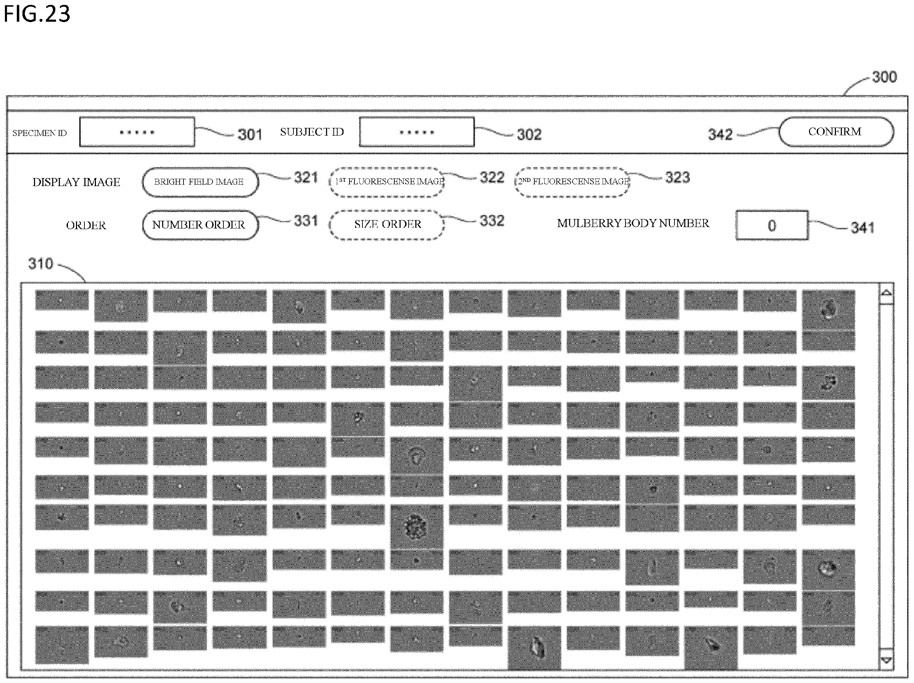

In step S7, the processor 11 causes the display unit 14 to display images of particles extracted through the third extraction step in step S6. Displaying of the images of the particles will be described later with reference to FIG. 23 to FIG. 28.

Next, the first to third extraction steps regarding mulberry bodies will be described with reference to FIG. 4 to FIG. 13.

Although the extraction steps are described below as steps of generating scattergrams 210,220,230 by processor 11 at the time of extraction as shown in FIGS. 4, 7, and 11, scattergrams do not necessarily have to be generated. That is, the scattergrams 210,220,230 are for convenience of description only and it is sufficient that particles whose a combination of two values for each of the particles is included in a range on data corresponding to the scattergram 210,220,230 is extracted through data process.

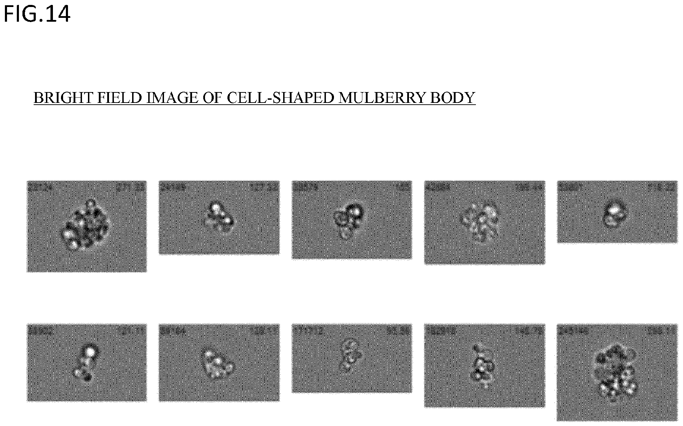

In the following scattergrams, plotted dots are color-coded on the basis of a result of determination as to whether or not the image stored in the storage 12 is a mulberry body through visual checking by a skilled laboratory technician. However, as described later with reference to FIG. 14, mulberry bodies include those having a cell shape. The laboratory technician individually determines mulberry bodies having cell shapes and mulberry bodies other than the cell shapes and determines that each of the mulberry bodies having the cell shape and the mulberry bodies other than the cell shape is a mulberry body. In the scattergrams shown below, unless otherwise specified, for convenience, the plotted gray dots indicate mulberry bodies having no cell shapes, and the plotted white dots indicate mulberry bodies having cell shapes and particles other than mulberry bodies. The colors of the plotted dots are for the purpose of checking the extraction step of mulberry bodies and may be omitted.

FIG. 4 shows the scattergram 210 generated in the first extraction step.

The processor 11 obtains a maximum luminance from the first fluorescence image corresponding to one particle and obtains a maximum luminance from the second fluorescence image corresponding to the particle. Specifically, the processor 11 extracts pixels corresponding to regions of particles from the first fluorescence images and obtains, as the maximum luminance, the luminance of the pixel having the largest luminance among the extracted pixels. The pixels corresponding to the regions of the particles may be extracted on the basis of change in luminances between adjacent pixels in the first fluorescence image, or may be extracted by using bright field images of identical particles. The processor 11 obtains such maximum luminances with respect to all the particles, i.e., all the first and second fluorescence images stored in the storage 12.

The maximum luminance obtained from the first fluorescence image is an example of a value related to an intensity of the fluorescence having the wavelength λ21 and generated from the particle, and the maximum luminance obtained from the second fluorescence image is an example of a value related to an intensity of the fluorescence having the wavelength λ22 and generated from the same particle.

Although the value related to the intensity of the fluorescence having the wavelength λ21 and generated from the particle is the maximum luminance in the first extraction step of the present embodiment, the value is not limited to the maximum luminance. For example, the value may be an average value, a total value, a median value, or the like of the luminances of the pixels identified as constituting the region of the particle in the first fluorescence image. Likewise, although the value related to the intensity of the fluorescence having the wavelength λ22 from the particle is the maximum luminance in the present embodiment, the value is not limited to the maximum luminance. For example, the value may be the average value, the total value, the median value, or the like of the luminances of the pixels identified as constituting the region of the particle in the second fluorescence image.

The processor 11 plots all the particles onto the scattergram 210 by using the maximum luminances obtained from the first and second fluorescence images. The scattergram 210 has a vertical axis indicating the maximum luminance having the wavelength λ21 (in the wavelength band of 435 nm or more and 505 nm or less), i.e., the maximum luminance based on the first fluorescence image. The scattergram 210 has a horizontal axis indicating the maximum luminance having the wavelength λ22 (in the wavelength band of 505 nm or more and 560 nm or less), i.e., the maximum luminance based on the second fluorescence image.

The processor 11 extracts, from the scattergram 210, particles included in a range R1 corresponding to mulberry bodies. The range R1 is a fixed range that is prestored in the storage 12.

A range R11 is a range corresponding to particles of a first type other than mulberry bodies. The first type of particle is, for example, a squamous epithelial cell, a tubular epithelial cell, or the like. A range R12 is a range corresponding to particles of a second type other than mulberry bodies. The second type of particle is, for example, a salt, a bacterium, or the like.

A range R1 is a range corresponding to mulberry bodies. The range R1 is set to a range in which the first type of particles distributed in the range R11 and the second type of particles distributed in the range R12 can be excluded. Specifically, the range R1 is a range that is narrow at and near the origin of the scattergram 210, widens as the values of the vertical axis and the horizontal axis of the scattergram 210 increase, and is sandwiched between: the range R11 corresponding to the particles of the first type other than mulberry bodies; and the range R12 corresponding to the particles of the second type other than mulberry bodies. The range R1 is a range sandwiched between a straight line R1a and a straight line R1b. The straight line R1a is a boundary between the range R1 and the range R11, and the straight line R1b is a boundary between the range R1 and the range R12. Specifically, for example, the straight line R1a is a straight line passing through the origin of the scattergram 210 and having a first angle with respect to the horizontal axis of the scattergram 210, and the straight line R1b is a straight line passing through the origin of the scattergram 210 and having a second angle different from the first angle with respect to the horizontal axis of the scattergram 210.

In the present embodiment, when the mulberry bodies are irradiated with the excitation light having the wavelength of 405 nm, the intensities of the autofluorescences having the wavelength λ21 and the autofluorescences having the wavelength λ22 generated from the mulberry bodies are approximately equal to each other. Meanwhile, when particles other than mulberry bodies are irradiated with the excitation light having the wavelength of 405 nm, the intensities of the fluorescences having the wavelength λ21 and the fluorescences having the wavelength λ22 generated from the other particles are, unlike mulberry bodies, different from each other. Therefore, as shown in FIG. 4, by setting the range R1 to be a range extending from the vicinity of the origin at an angle of about 45° to the two axes of the scattergram 210 and setting the ranges R11 and R12 outside the range R1, mulberry bodies can be accurately distinguished from the other particles. Upon extraction of the particles in the range R1, the first extraction step is ended.

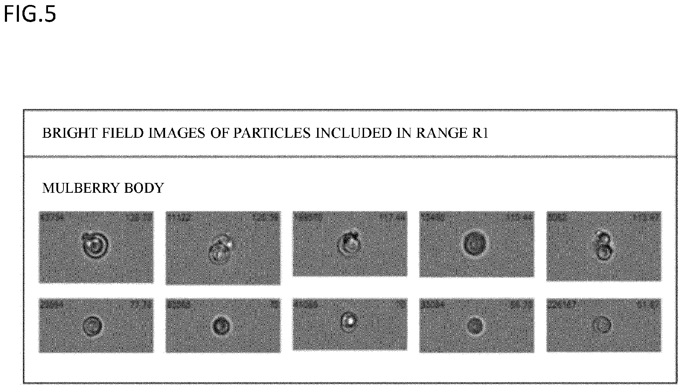

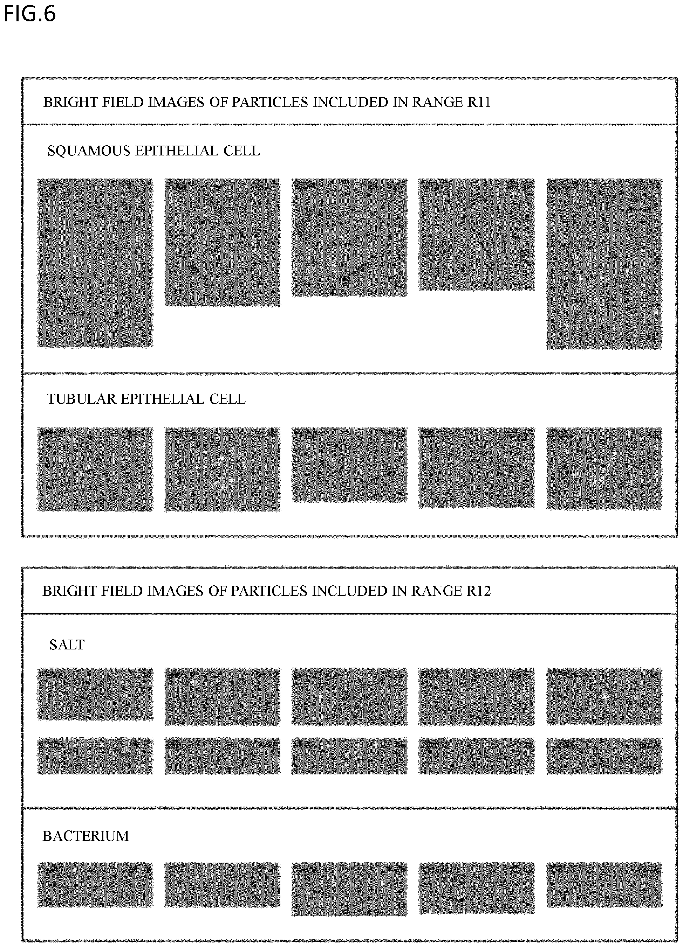

FIG. 5 shows bright field images of particles included in the range R1. FIG. 6 shows bright field images of particles included in the ranges R11 and R12.

The particles included in the range R1 are, for example, mulberry bodies shown in FIG. 5. The first type of particle included in the range R11 is, for example, a squamous epithelial cell, a tubular epithelial cell, or the like shown on the upper stage of FIG. 6. The second type of particle included in the range R12 is, for example, a salt, a bacterium, or the like shown on the lower stage of FIG. 6.

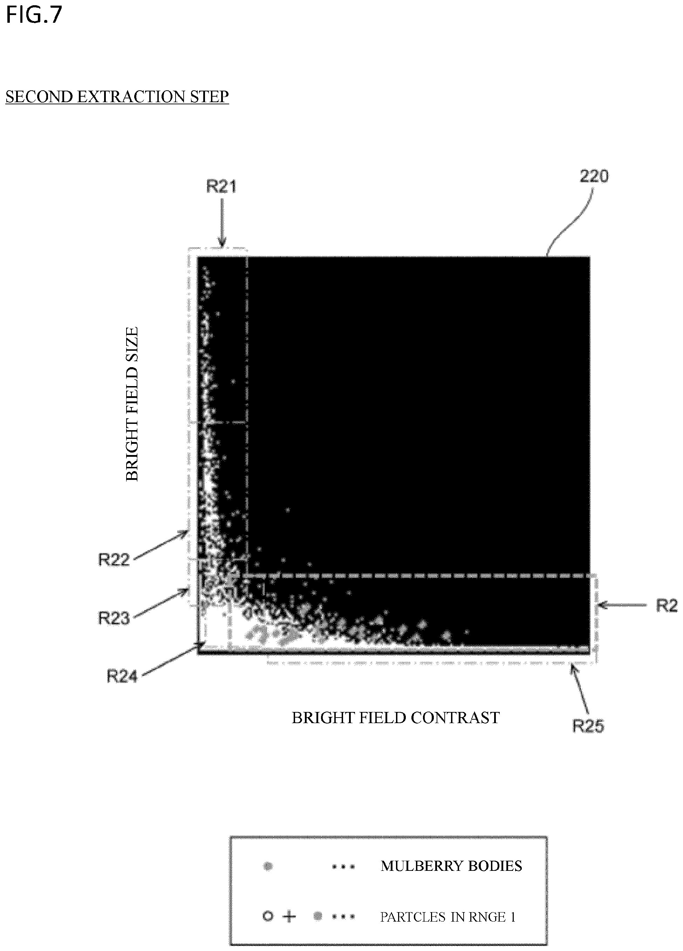

FIG. 7 shows the scattergram 220 generated in the second extraction step.

The processor 11 obtains a bright field size and a bright field contrast from the bright field image of each of the particles in the range R1 extracted through the first extraction step.

The bright field size is an example of a value related to a size of the corresponding particle based on the bright field image and is morphological information about the particle. For example, the processor 11 extracts pixels corresponding to a particles from the bright field image and obtains a number of the extracted pixels as the bright field size. The pixel corresponding to the particle may be extracted on the basis of a change in luminance between adjacent pixels in the bright field image.

The bright field contrast is an example of a value related to a contrast of the corresponding particle based on the bright field image and is morphological information about the particle. For example, the processor 11 obtains, as the bright field contrast, a value obtained by: within pixels identified as corresponding to a particle in the bright field image, adding up gradients of luminances in a pixel range composed of 3 by 3 pixels and performing shifts to different pixel ranges; calculating gradients of luminances in the respective pixel ranges; and dividing the total gradient of the luminances of all the pixel ranges by the number of pixels in the region of the particle. The bright field contrast indicates an amount of changes in the luminance in the bright field image. For example, in the bright field image, a larger difference in darkness/paleness between the edge and the inside of the particle leads to a larger bright field contrast. The bright field contrast varies according to the difference in darkness/paleness between the aggregate surface and the inside, the difference in darkness/paleness due to the shapes of spirals of mulberry bodies, or the like.

The processor 11 plots all the particles in the range R1 extracted through the first extraction step onto the scattergram 220 by using the bright field sizes and the bright field contrasts obtained from the bright field images. The scattergram 220 has a horizontal axis indicating the bright field contrast and a vertical axis indicating the bright field size.

The processor 11 extracts, from the scattergram 220, particles included in a range R2 corresponding to mulberry bodies. The range R2 is a fixed range that is prestored in the storage 12.

The ranges R21 and R22 are ranges corresponding to squamous epithelial cells. A range R23 is a range corresponding to urothelial cells. A range R24 is a range corresponding to tubular epithelial cells, white blood cells, and red blood cells. A range R25 is a range corresponding to salts and bacteria.

A range R2 is a range corresponding to mulberry bodies. The range R2 is set to a range in which squamous epithelial cells distributed in and near the ranges R21 and R22, urothelial cells distributed in and near the range R23, tubular epithelial cells, white blood cells, and red blood cells distributed in and near the range R24, and salts and bacteria distributed in and near the range R25 can be excluded. Specifically, the range R2 is set to a range in which: the bright field contrast is approximately larger than that in the ranges R21 to R24 in which the squamous epithelial cells, the urothelial cells, the tubular epithelial cells, the white blood cells, the red blood cells, and the like are distributed; and the bright field size is larger than that in the range R25 in which small particles such as salts and bacteria are distributed. Upon extraction of the particles in the range R2, the second extraction step is ended.

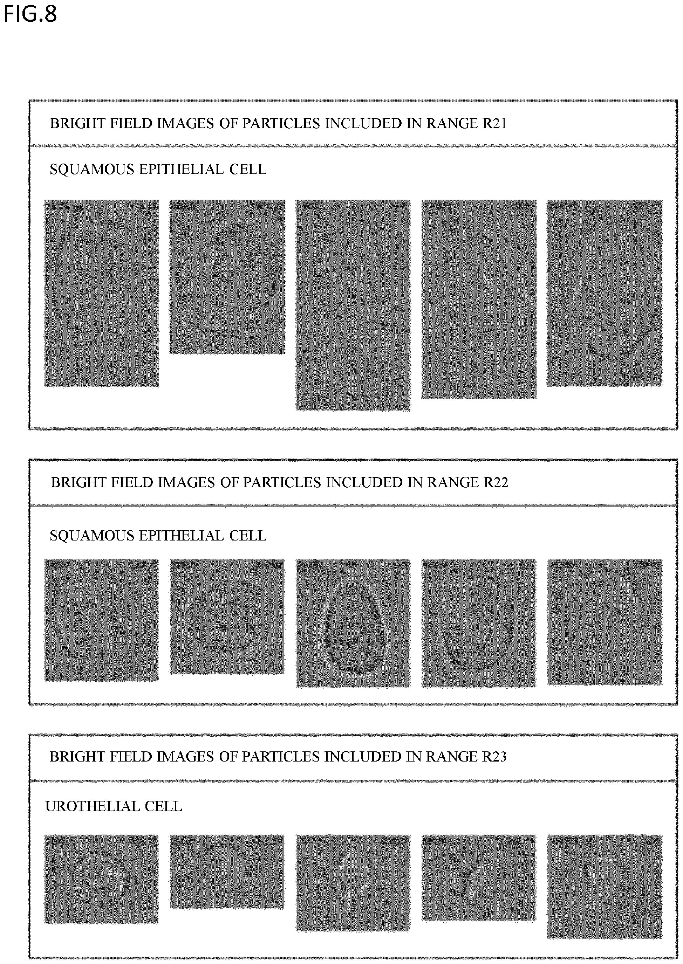

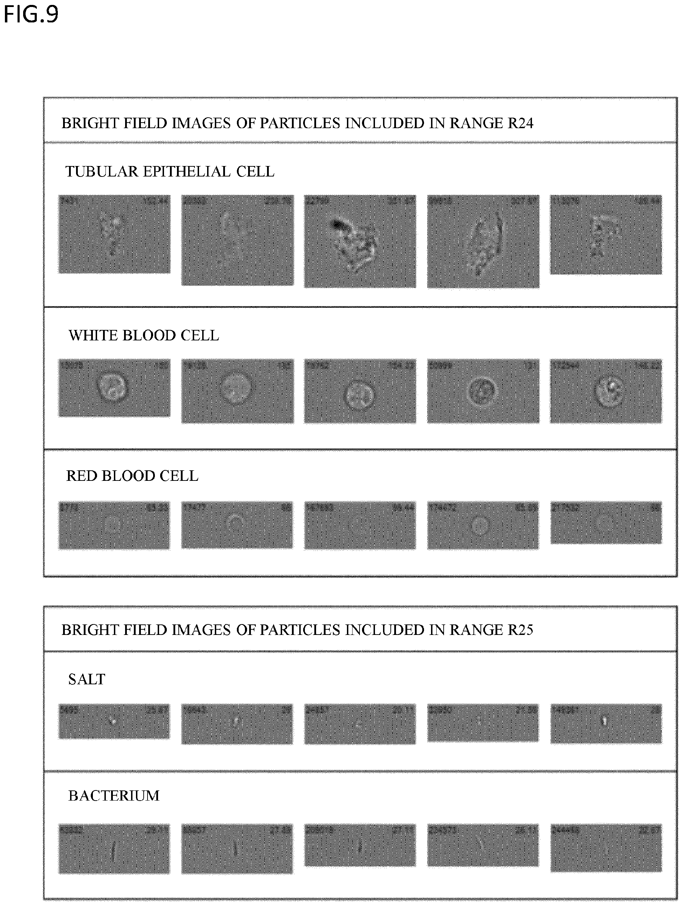

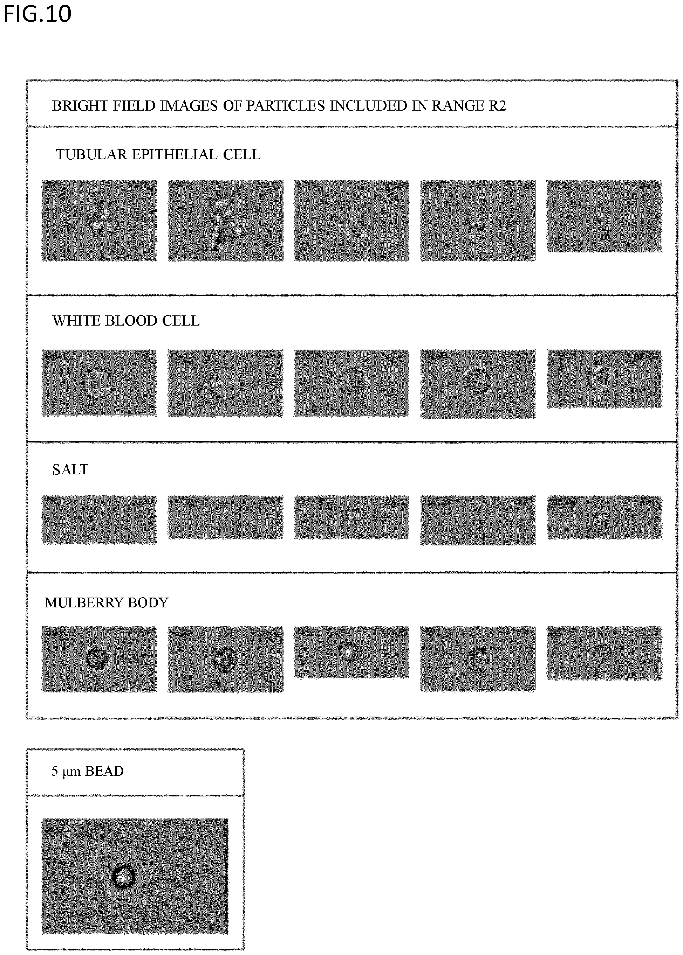

FIG. 8 shows bright field images of particles included in the range R21,R22,R23. FIG. 9 shows bright field images of particles included in the ranges R24 and R25. FIG. 10 shows bright field images of particles included in the range R2.

The particles included in the range R21 are, for example, squamous epithelial cells shown on the upper stage of FIG. 8, and the like. The particles included in the range R22 are, for example, squamous epithelial cells shown on the intermediate stage of FIG. 8, and the like. The particles included in the range R23 are, for example, urothelial cells shown on the lower stage of FIG. 8, and the like. The squamous epithelial cells and the urothelial cells in the ranges R21 to R23 shown in FIG. 8 have sizes that are approximately larger than those of mulberry bodies and have bright field contrasts that are approximately smaller than those of mulberry bodies. Since such particles have a low probability of being mulberry bodies, particles distributed in the ranges R21 to R23 can be excluded through the second extraction step.

The particles included in the range R24 are, for example, tubular epithelial cells, white blood cells, red blood cells, and the like shown on the upper stage of FIG. 9. The tubular epithelial cells, the white blood cells, and the red blood cells in the range R24 shown on the upper stage of FIG. 9 have bright field contrasts approximately smaller than those of mulberry bodies. In particular, since red blood cells in the bright field images are shown as ones having circular shapes and are morphologically similar to mulberry bodies, it is difficult to distinguish mulberry bodies and red blood cells from each other through visual checking of the bright field images. However, red blood cells have smaller bright field contrasts than mulberry bodies. Since the particles having the small bright field contrasts have a low probability of being mulberry bodies as described above, the particles distributed in the range R24 can be excluded through the second extraction step.

The particles included in the range R25 are, for example, salts, bacteria, and the like shown on the lower stage of FIG. 9. The salts and bacteria in the range R25 shown on the lower stage of FIG. 9 have sizes that are much smaller than the sizes of mulberry bodies. Since such particles have a low probability of being mulberry bodies, the particles distributed in the range R25 can be excluded through the second extraction step.

The particles included in the range R2 are, for example, tubular epithelial cells, white blood cells, salts, mulberry bodies, and the like shown on the upper stage of FIG. 10. In the bright field images of mulberry bodies in the range R2 shown on the upper stage of FIG. 10, spirals and the like are formed inside. The tubular epithelial cells, white blood cells, salts, and the like in the range R2 shown on the upper stage of FIG. 10 have sizes approximately equal to those of mulberry bodies, and have bright field contrasts approximately equal to those of mulberry bodies. Therefore, when the particles distributed in the range R2 are extracted through the second extraction step, the extracted particles also include particles other than mulberry bodies as shown on the upper stage of FIG. 10.

In addition, a bright field image obtained by imaging a bead having a size of 5 µm that is approximately equal to the size of a mulberry body by the particle detection apparatus 1 is shown on the lower stage of FIG. 10. When the range R2 is preset, the ranges of bright field sizes and bright field contrasts of the range R2 can be set by using, for example, bright field images obtained by imaging beads having predetermined sizes in this manner.

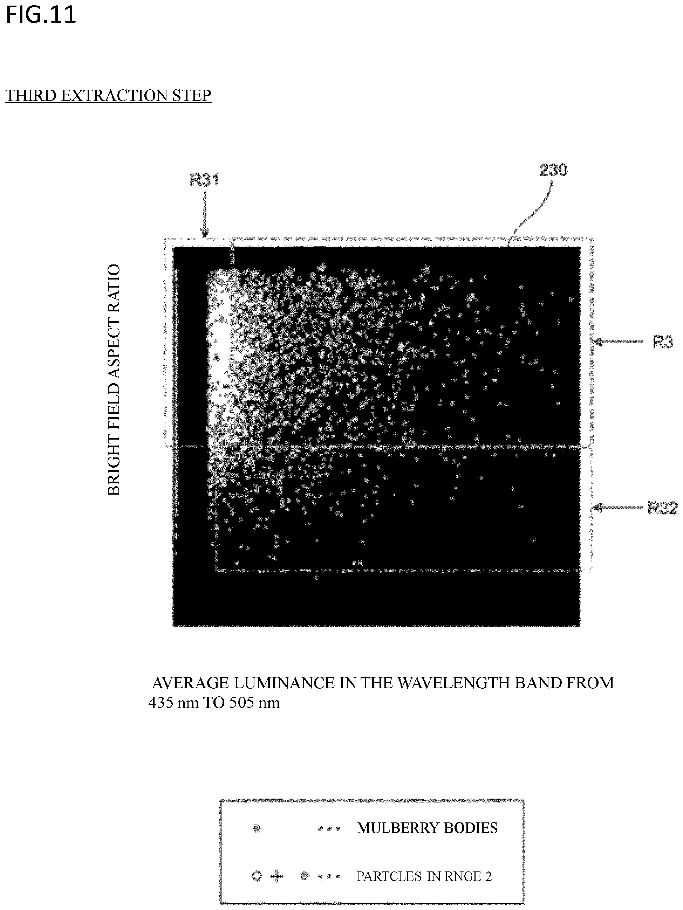

FIG. 11 shows the scattergram 230 generated in the third extraction step

The processor 11 obtains a bright field aspect ratio from the bright field image of each of the particles in the range R2 extracted through the second extraction step, and obtains an average luminance from the first fluorescence image of each of the particles in the range R2 extracted through the second extraction step.

The bright field aspect ratio is an example of a value related to a shape of a particle based on the bright field image and is morphological information about the particle. For example, the processor 11 obtains, as the bright field aspect ratio, an aspect ratio of each of groups of pixels identified as corresponding to the particle in the bright field image.

The average luminance obtained from the first fluorescence image is an example of a value related to an intensity of the fluorescence having the wavelength λ21 and generated from a particle. For example, the processor 11 obtains, as the average luminance, an average value of the luminances of the pixels identified as constituting a region of the particle in the first fluorescence image.

Although the value related to the intensity of the fluorescence having the wavelength λ21 and generated from the particle is the average luminance in the third extraction step of the present embodiment, the value is not limited to the average luminance. For example, the value may be the maximum value, the total value, the median value, or the like of the luminances of the pixels identified as constituting the region of the particle in the first fluorescence image.

The processor 11 plots all the particles in the range R2 extracted through the second extraction step onto the scattergram 230 by using the bright field aspect ratio obtained from the bright field image and the average luminance obtained from the first fluorescence image. The scattergram 230 has a vertical axis indicating the bright field aspect ratio. As the value of the vertical axis increases and proceeds upward, the value of the bright field aspect ratio approaches 1. The scattergram 210 has a horizontal axis indicating the average luminance having the wavelength λ21 (in the wavelength band of 435 nm or more and 505 nm or less), i.e., the average luminance based on the first fluorescence image.

The processor 11 extracts, from the scattergram 230, particles included in a range R3 corresponding to mulberry bodies. The range R3 is a fixed range that is prestored in the storage 12.

A range R31 is a range corresponding to tubular epithelial cells, salts, fat globules, crystals, and bacteria. A range R32 is a range corresponding to particles such as cylinders.

The range R3 is a range corresponding to mulberry bodies. The range R3 is set to a range in which tubular epithelial cells, salts, fat globules, crystals, and bacteria distributed in and near the range R31, and particles such as cylinders distributed in and near the range R32 can be excluded. Specifically, the range R3 is set to a range in which: the average luminance at the wavelength λ21 (in the wavelength band of 435 nm or more and 505 nm or less), i.e., the average luminance based on the first fluorescence image is larger than that in the range R31 in which the tubular epithelial cells, the salts, the fat globules, the crystals, and the bacteria are distributed; and the bright field aspect ratio is larger than that in the range R32 in which the particles such as the cylinders are distributed. Upon extraction of the particles in the range R3, the third extraction step is ended.

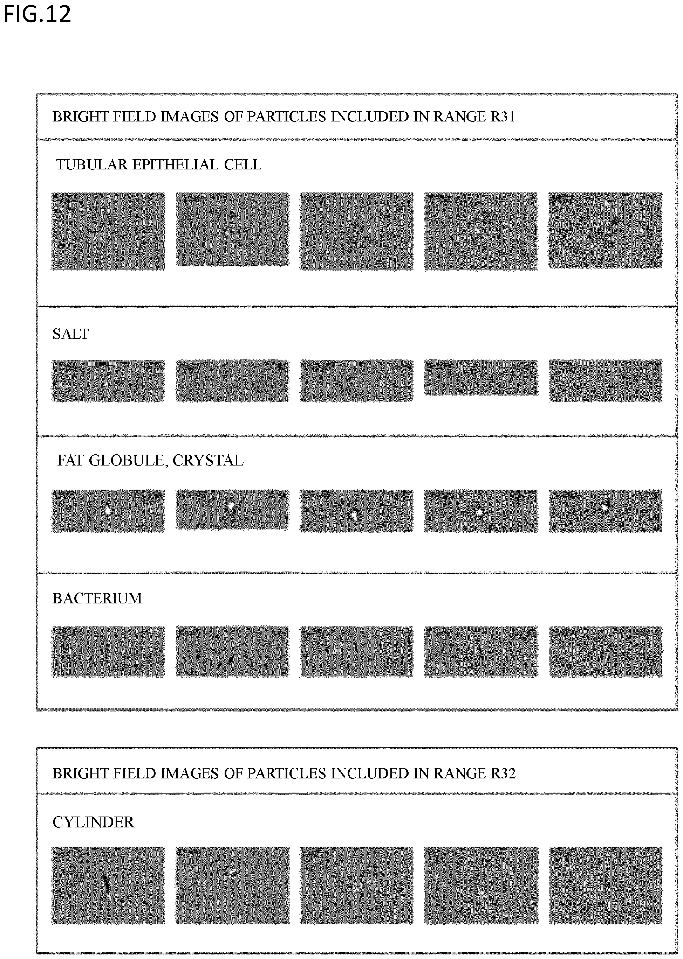

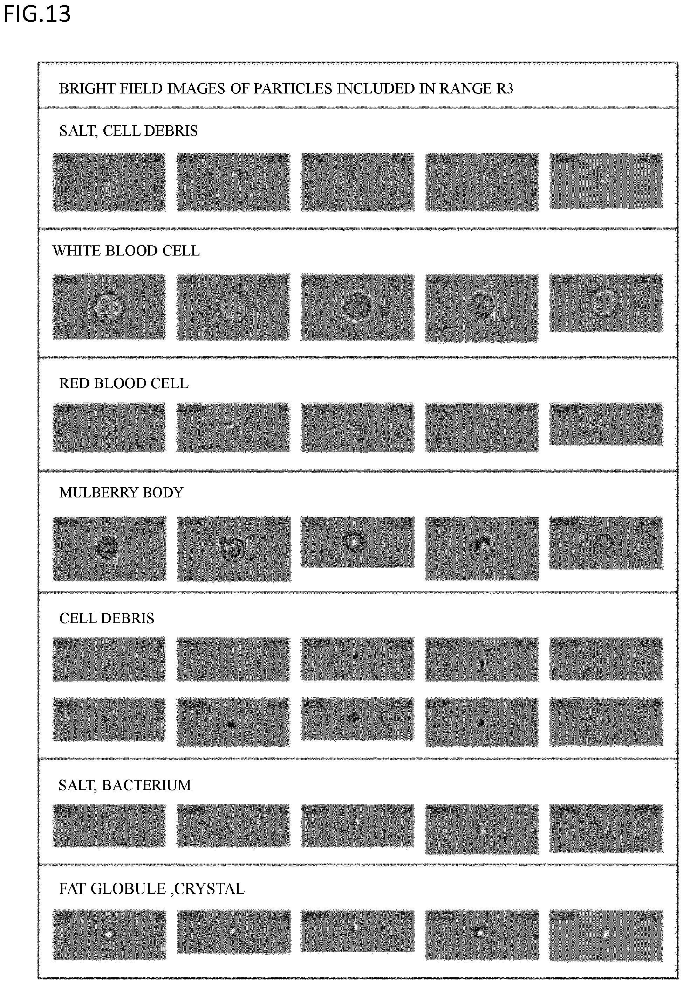

FIG. 12 is a diagram showing bright-field images of particles included in ranges R31 and R32. FIG. 13 is a diagram showing a bright-field image of particles included in the range R3 .

The particles included in the range R31 include, for example, tubular epithelial cells, salts, fat globules, crystals, and bacteria, which are shown in the upper part of FIG. 12. The particles included in the range R31 have a smaller average luminance at wavelength *λ* 21 (wavelength band of 435 nm or more and 505 nm or less) than that of mulberry bodies. Since such particles are unlikely to be mulberry bodies, the third extraction step can exclude particles distributed in range R31.

The particles included in the range R32 include, for example, cylinders shown in the lower part of FIG. 12. The particles included in the range R32 have a smaller bright field aspect ratio than that of mulberry bodies. Since such particles are unlikely to be mulberry bodies, the third extraction step can exclude particles distributed in the range R32.

The particles included in the range R3 include, for example, salts, cell debris, white blood cells, red blood cells, mulberry bodies, bacteria, fat globules, and crystals shown in FIG. 13. The particles included in the range R3 have average luminance and bright field aspect ratio at wavelength *λ* 21 (wavelength band of 435 nm or more and 505 nm or less) that are comparable to those of mulberry bodies. Therefore, when the particles distributed in the range R3 are extracted by the third extraction step, the extracted particles also include particles other than mulberry bodies as shown in FIG. 13.

In this way, particles other than mulberry bodies remain in the range R3, as shown in Figure 13, but the mulberry bodies contained in the range R3 can be easily identified by the operator visually using a screen 300 displayed on the display unit 14. The screen 300 will be described later with reference to FIG. 23 etc. According to the first to third extraction steps, by extracting particles contained in the ranges R1, R2, R3 corresponding to mulberry bodies, many particles other than mulberry bodies contained in the urine specimen are excluded, allowing the operator to accurately distinguish mulberry bodies from other particles by referring to images displayed on the screen 300 described below.

Next, we will explain mulberry bodies, which have a cellular shape.

FIG. 14 shows a bright field image of a cell-shaped mulberry body. Mulberry bodies, which have this type of cell shape, are sometimes called mulberry cells. The cell-shaped mulberry bodies may include conglomerate (mulberry-like) shapes. Even when cell-shaped mulberry bodies are contained in a urine specimen, the cell-shaped mulberry bodies are discriminated from other particles other than mulberry bodies by using ranges similar to the ranges R1, R2, and R3 in the first to third extraction steps described above.

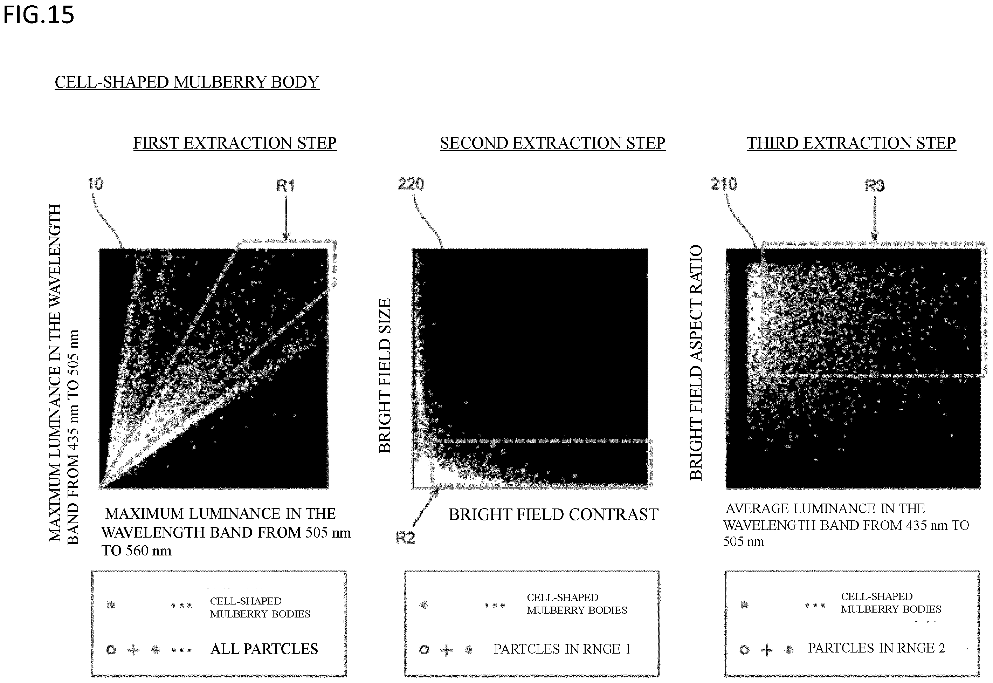

FIG. 15 is a diagram showing discrimination of cell-shaped mulberry bodies contained in a urine specimen.

The scattergrams 210, 220, and 230 shown in FIG. 15 are generated from the same images used to generate the scattergrams 210, 220, and 230 shown in FIGS. 4, 7, and 11, respectively. In the scattergrams 210, 220, and 230 of FIG. 15, the gray plots indicate cell-shaped mulberry bodies, and the white plots indicate mulberry bodies having no cellular shape and particles other than mulberry bodies.

As shown in FIG. 15, cell-shaped mulberry bodies are included in the ranges R1, R2, and R3, similar to FIGs. 4, 7, and 11. Therefore, by setting ranges similar to the ranges R1, R2, and R3 in the first to third extraction steps, it is possible to distinguish cell-shaped mulberry bodies from other particles other than mulberry bodies. Therefore, by carrying out the first to third extraction steps in the same manner as described above, cell-shaped mulberry bodies and mulberry bodies not having a cellular shape can be distinguished from particles other than mulberry bodies.

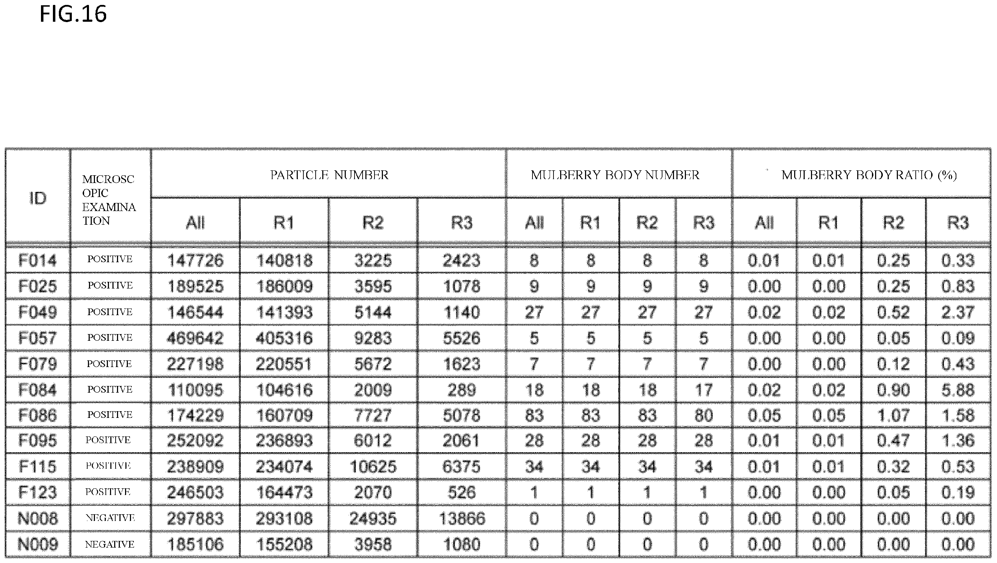

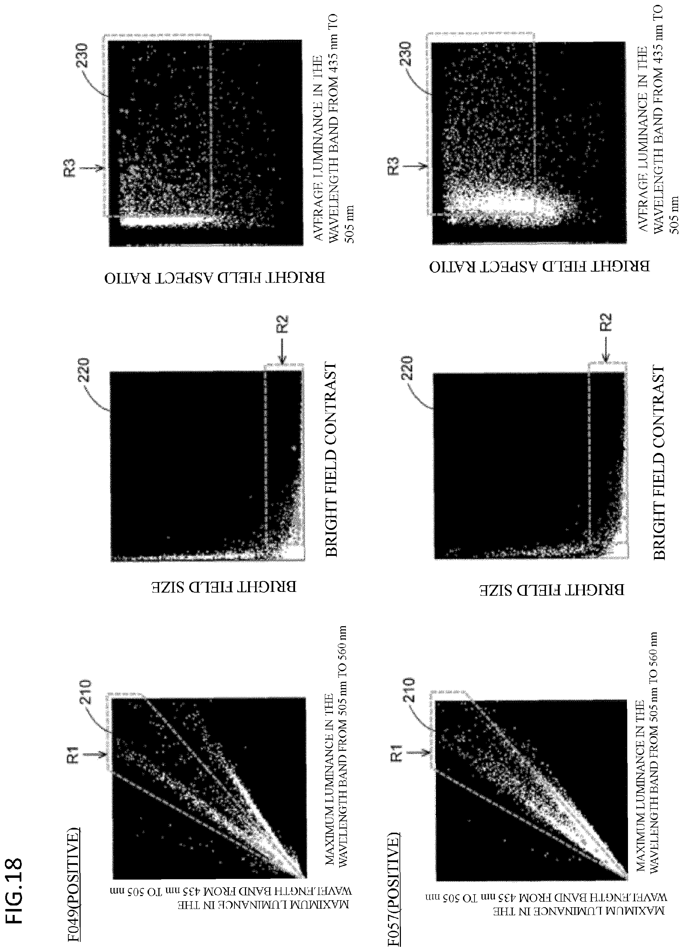

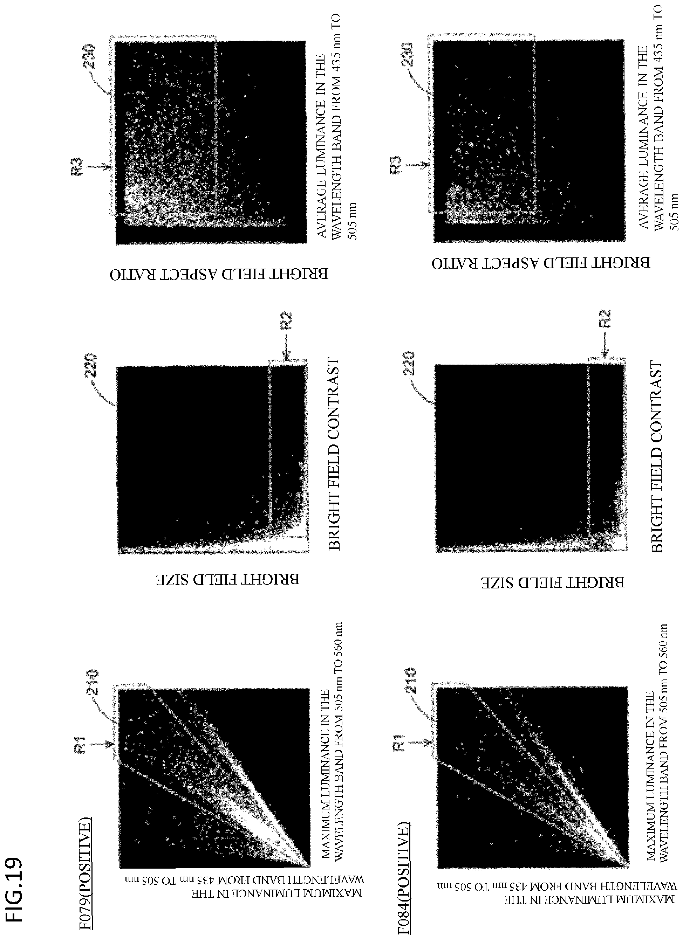

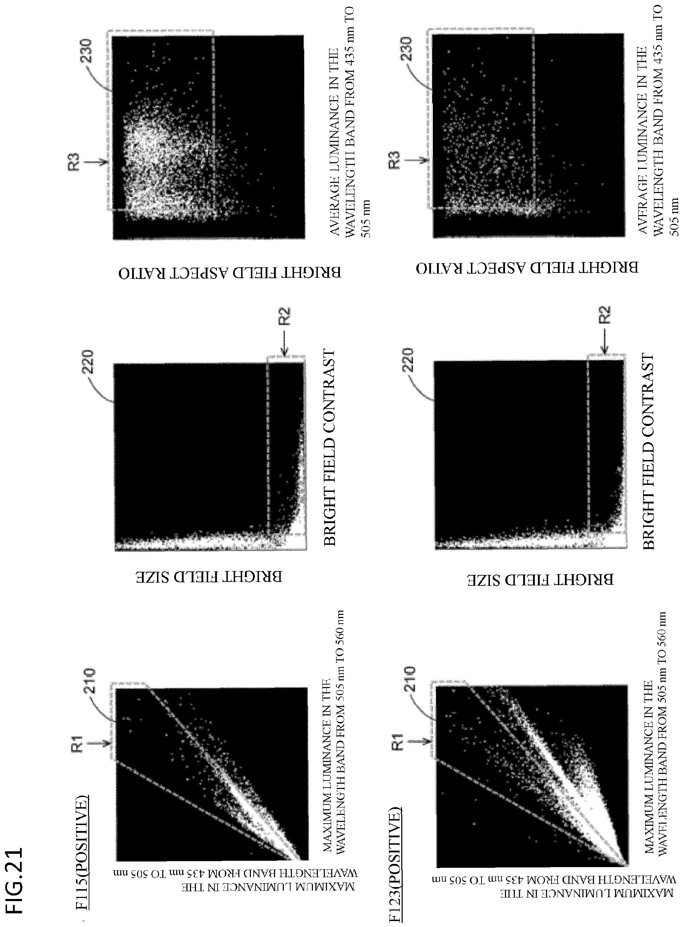

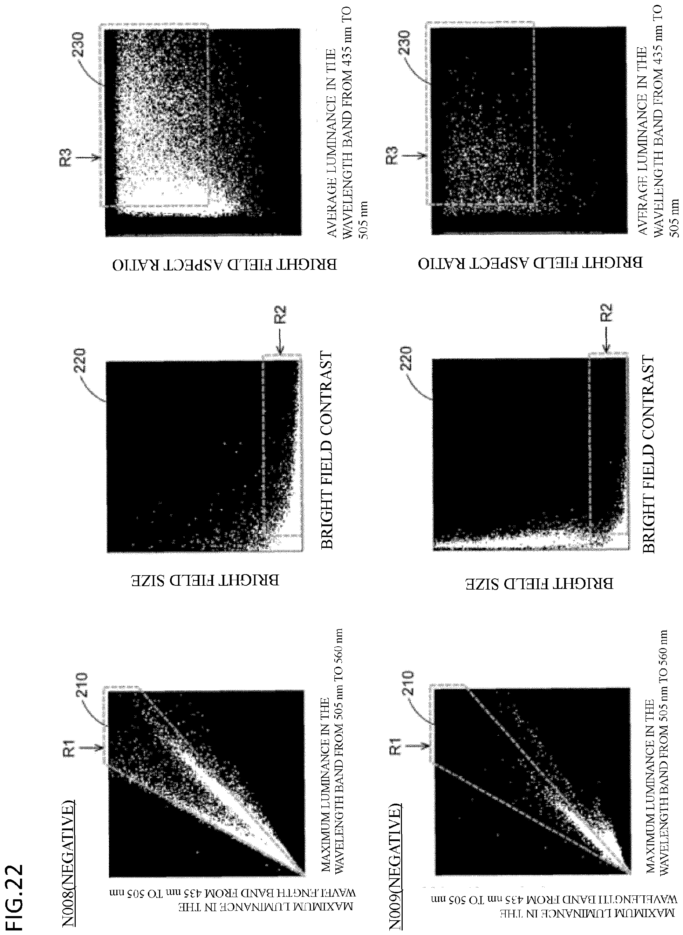

Next, with reference to Figs. 16 to 22, experiments in which the first to third extraction steps were carried out on 12 subjects to extract mulberry bodies will be described.

FIG. 16 is a table showing a number of particles, a number of mulberry bodies, and a proportion of mulberry bodies contained in each range in the first to third extraction steps.

In this experiment, urine specimens were collected from each of 12 subjects, some of the collected urine specimens were subjected to microscopic examination by an experienced laboratory technician, and the remaining specimens were subjected to the processing in steps S1 to S6 of FIG. 3.

The laboratory technician performed a microscopic examination on a portion of the urine specimen, and if the technician determined that the urine specimen contained mulberry bodies, the urine specimen was deemed positive, and if the technician determined that the urine specimen did not contain mulberry bodies, the urine specimen was deemed negative. The laboratory technician determined that both cellular-shaped and non-cellular-shaped mulberry bodies were mulberry bodies. Urine specimens with specimen IDs F014, F025, F049, F057, F079, F084, F086, F095, F 115, and F 123 were determined to be positive by microscopic examination. The urine specimens with specimen IDs N008 and N009 were determined to be negative based on the microscopic results.

On the other hand, for another portion of the same urine specimens, steps S1 to S6 in FIG. 3 were carried out to obtain a number of particles, a number of mulberry bodies, and a ratio of mulberry bodies in the ranges R1, R2, and R3. Whether or not a particle was a mulberry body was judged by a skilled technician by visually inspecting images stored in the storage 12. Again, the technician determined that both cellular-shaped and non-cellular-shaped mulberry bodies were mulberry bodies.

"All" in the column of particles in the table indicates a number of particles contained in the scattergram 210 . "R1", "R2", and "R3" in the columns of the particles in the table are numbers of particles included in the ranges R1, R2, and R3 of the scattergram 210, respectively. "All" in the column of the number of Mulberry bodies in the table is a number of mulberry bodies contained in the scattergram 210 . "R1", "R2", and "R3" in the column of the number of mulberry bodies in the table are numbers of mulberry bodies included in the ranges R1, R2, and R3 of the scattergram 210, respectively. "All" in the column of the ratio of mulberry bodies in the table is a ratio of the number of mulberry bodies to the number of particles contained in the scattergram 210 . "R1", "R2", and "R3" in the column of the ratio of mulberry bodies in the table respectively represent ratios of the number of mulberry bodies to the number of particles contained in the ranges R1, R2, and R3 of the scattergram 210.

As shown in FIG. 16, by carrying out the first to third extraction steps, the number of particles was significantly reduced in all urine specimens. On the other hand, in all urine specimens, the number of mulberry bodies was hardly decreased by the first to third extraction steps. In other words, the number of mulberry bodies finally extracted by the range R3 was only slightly reduced from all the mulberry bodies corresponding to the images stored in the storage 12. As a result, the ratio of mulberry bodies increased with each extraction, eventually increasing significantly. Therefore, it can be seen that mulberry bodies could be more accurately distinguished from all particles in the urine specimen.

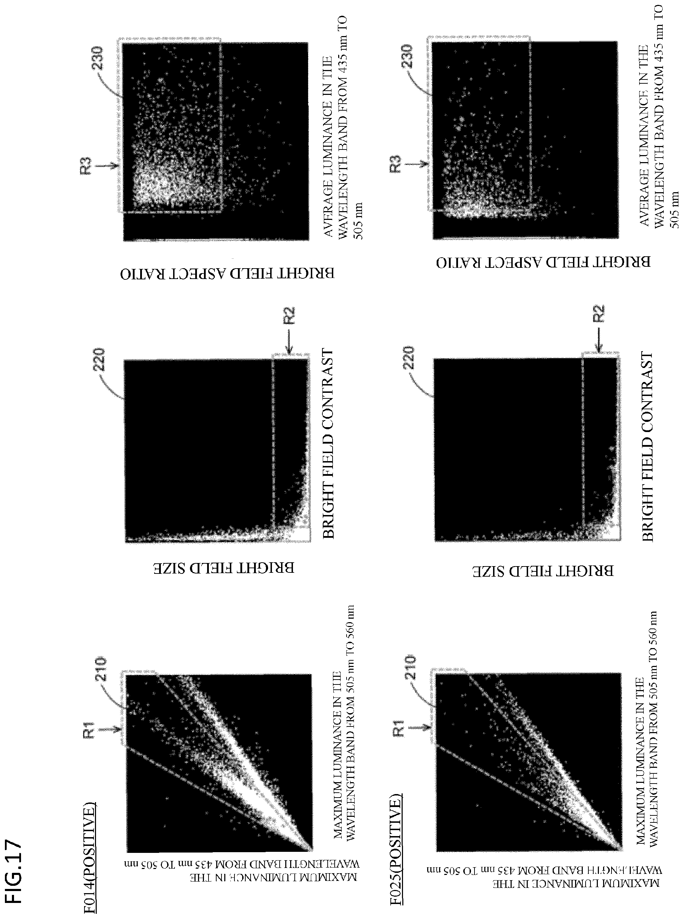

Figs. 17 to 22 show scattergrams 210, 220, and 230 obtained in the first to third extraction steps for the urine specimen shown in FIG. 16.

Also in Figures 17 to 22, the gray plots correspond to particles that are determined to be mulberry bodies not having a cellular shape by an experienced technician visually inspecting the image captured by the imaging unit 20 in step S3, and the white plots correspond to particles that are determined to be cell-shaped mulberry bodies or particles other than mulberry bodies.

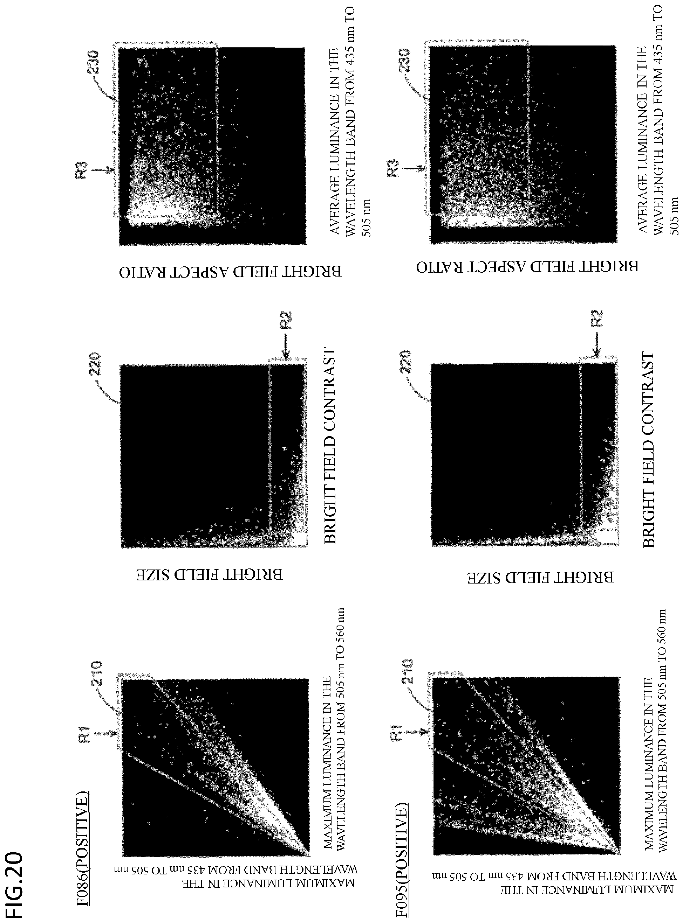

Moreover, the urine specimen with the specimen ID F095 is the same as the urine specimen exemplified in the explanation of Figs. 4 to 15. Therefore, the scattergrams 210, 220, and 230 shown in the lower part of FIG. 20 are the same as the scattergrams shown in Figs. 4, 7, and 11.

As can be seen by referring to the scattergrams 210, 220, and 230 of the urine specimens in Figures 17 to 22, plots of mulberry bodies not having a cellular shape, shown in gray, are approximately within the ranges R1, R2, and R3 of the first to third extraction steps. Furthermore, as shown with reference to Figure 15, mulberry bodies having a cellular shape are distributed in approximately the same positions as mulberry bodies having no cellular shape, and can be said to be approximately within the ranges R1, R2, and R3 of the first to third extraction steps. In this way, it can be seen that by using the ranges R1, R2, and R3 pre-stored in the storage 12, mulberry bodies can be accurately distinguished for any urine specimens.

Next, the process of displaying images of particles, which is performed in step S7 of FIG. 3, will be described with reference to Figs. 23 to 28. In step S7 of FIG. 3, images of the particles extracted by the first to third extraction processes (the particles included in range R3 of scattergram 230 in FIG. 11) are displayed on a screen 300 shown below, and history of mulberry body counting results and information based on changes in the counting results are displayed on a screen 400 shown below.

FIG. 23 is a diagram showing a schematic configuration of the screen 300 for displaying images of particles.

The screen 300 includes a specimen ID display area 301, a subject ID display area 302, and an image display area 310 for displaying images of particles. The screen 300 also includes a bright field image display button 321, a first fluorescent image display button 322, and a second fluorescent image display button 323 for selecting a type of images to be displayed in the image display area 310. The screen 300 also includes a number order button 331 and a size order button 332 for setting an order of images to be displayed in the image display area 310 . The screen 300 also includes a counting result display area 341 that displays a number of mulberry bodies selected in the image display area 310, and a confirm button 342 for confirming the counting result.

When an operator inputs a specimen ID and a subject ID and then inputs an instruction to display the screen 300, the processor 11 displays the screen 300 shown in FIG. 23 on the display unit 14.

When the operator operates any of the bright field image display button 321, the first fluorescent image display button 322, or the second fluorescent image display button 323, the processor 11 displays images in a type corresponding to the operated button in the image display area 310. In the image display area 310, images of particles contained in the range R3 obtained from the urine specimen indicated in the specimen ID display area 301 is displayed. The example shown in FIG. 23 shows a state in which the bright field image display button 321 out of the three buttons for selecting the type of image is operated. As a result, bright field images are displayed in the image display area 310.

When the operator operates either the number order button 331 or the size order button 332, the processor 11 changes an order of the images displayed in the image display area 310 . Each image is assigned a serial number of the particles, and when the number order button 331 is operated, the images are rearranged in the order of the serial numbers. When the size order button 332 is operated, the images are sorted in order of the size of the particles on the image based on the bright field size acquired from the bright field image. The example shown in FIG. 23 shows a state in which the number order button 331, one of the two buttons for setting the sorting order, has been operated. In both the number order button 331 and the size order button 332, the display order is switched between ascending order and descending order by successively operating the same button. In the example shown in FIG. 23, since no mulberry bodies have been selected (to be described later), "0" is displayed in the count result display area 341.

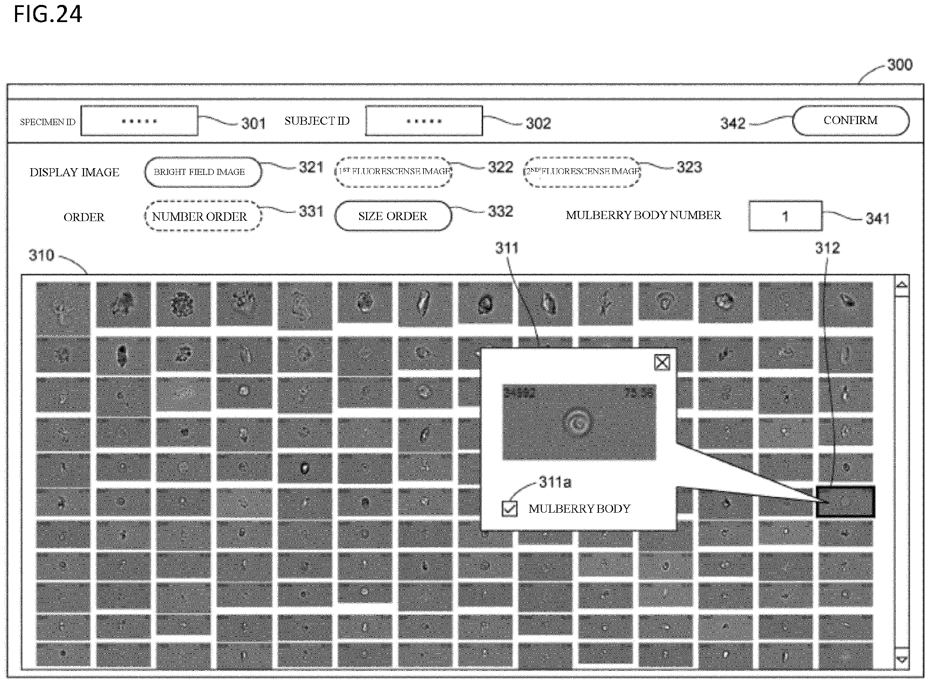

The example shown in FIG. 24 shows a state in which the size order button 332 of the two buttons for setting the sorting order has been operated. Since mulberry bodies are of comparable size, arranging the images in order of size results in images corresponding to mulberry bodies being arranged closely together. This allows the operator to select mulberry bodies more smoothly and accurately.

When the screen 300 is displayed, the processor 11 may initially display the images in the image display area 310 in serial number order or in size order. In addition, screen 300 may be provided with at least one of a button for sorting images in an order of the values of each axis obtained in the first extraction process, a button for sorting images in an order of the values of each axis obtained in the second extraction process, and a button for sorting images in an order of the values of each axis obtained in the third extraction process.

The operator refers to the images in the image display area 310, and when he or she determines that the particle shown in the image is highly likely to be a mulberry body, performs an operation on the image (for example, clicks on it). When the image in the image display area 310 is operated, the processor 11 displays an enlarged image area 311 superimposed on the screen 300 as shown in FIG. 24.

The enlarged image area 311 displays an enlarged image operated in the image display area 310. The enlarged image area 311 includes a check box 311a. The operator further observes the enlarged image, and if he or she determines that the target particle shown in the image is a mulberry body, he or she checks the check box 311a. When the checkbox 311a is checked, the processor 11 associates a flag with the bright field image, the first fluorescent image, and the second fluorescent image corresponding to the target particle and stores them in the storage 12 (see Figure 1), and adds a thick border 312 to the image of the target particle displayed in the image display area 310. When the image is thus given the thick border 312, it becomes easier to identify the images of particles determined to be mulberry bodies among multiple images in the image display area 310. In addition, when a flag is linked to the image of a checked particle, the image of the particle determined to be a mulberry body can be reviewed again at a later date.

The processor 11 displays a number of particles for which the check boxes 311a are checked in the count result display area 341 . This allows the operator to easily grasp the number of particles determined to be mulberry bodies (the number of particles marked with the thick border 312).

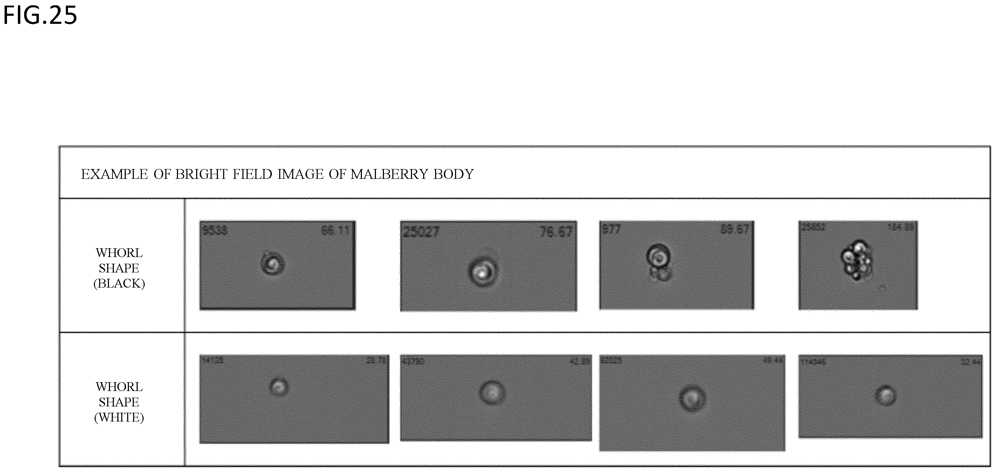

FIG. 25 illustrates examples of bright-field images of particles that were determined by the operator to be mulberry bodies.

Mulberry bodies may have a black whorl shape as shown in the first row, or a white whorl shape as shown in the second row. After grasping shapes of such mulberry bodies, the operator refers to the image display area 310 and the enlarged image area 311 shown in Figures 23 and 24 to determine whether the particle shown in the image is a mulberry body. At this time, as described above, since particles other than mulberry bodies have been largely removed by the first to third extraction steps, the operator can more smoothly and accurately perform the task of successively determining whether or not there are mulberry bodies by referring to images such as those shown in Figures 23 and 24.

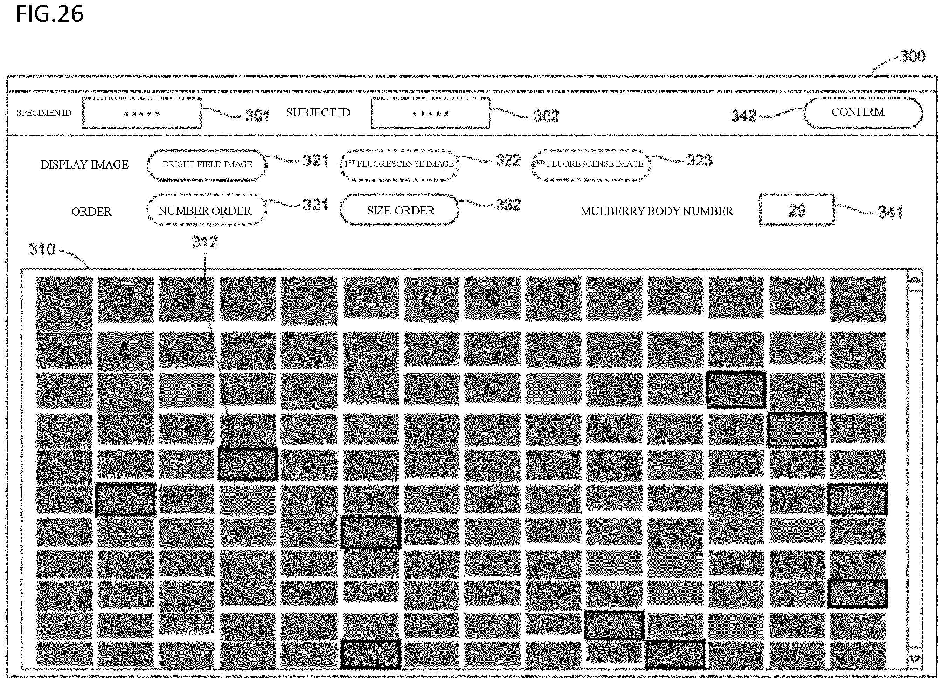

FIG. 26 is a diagram showing a schematic configuration of the screen 300 in a state where the operator has finished selecting mulberry bodies.

In the example shown in FIG. 26, the thick borders 312 are added to a plurality of images corresponding to mulberry bodies in the image display area 310 in response to the selection of the mulberry bodies by the operator. In addition, depending on the selection of mulberry bodies, the number of mulberry bodies is displayed in the count result display area 341.

When the operator has finished selecting the mulberry bodies, he or she operates the confirm button 342. As a result, the processor 11 transmits the counting results, including the number of particles determined to be mulberry bodies via the checkbox 311a, the ratio of the number of particles determined to be mulberry bodies to the number of particles within range R3, the specimen ID, the subject ID, and the current date and time, to an external host computer (see Figure 1). The counting results may be stored in the storage 12 instead of or in addition to being transmitted to the host computer.

In FIG. 24, only the enlarged image of the image operated within the image display area 310 is displayed in the enlarged image area 311; however, this is not limited to the above, and all images (bright field image, first fluorescent image, and second fluorescent image) corresponding to the particles in the image operated within the image display area 310 may be displayed. In this case, the operator can smoothly determine whether or not the target particle is a mulberry body while referring to all the images.

Additionally, in the enlarged image area 311, a check box for shapes of mulberry bodies (such as a spiral shape) may be provided. In this case, the operator refers to the image, judges the shape of the mulberry body, and checks the check box according to the shape of the mulberry body. The counting results for each shape of the mulberry bodies are displayed on the screen 300, and buttons for sorting the images by the shape of the mulberry bodies are provided. This makes it easier to identify mulberry bodies with typical shapes (such as whorls) and mulberry bodies with atypical shapes (other shapes) in the image display area 310, making it possible to confirm, for example, effectiveness of enzyme replacement therapy administered to a patient with Fabry disease.

In addition, the input of instructions to associate an image in the image display area 310 with a mulberry body is not limited to being made via the check box 311a, but may also be made via other operations on the image (e.g., right-clicking or double-clicking).

Furthermore, the scattergrams 210, 220, and 230 shown in Figs 4, 7, and 11 may be displayed on the screen 300. In this case, when the operator manipulates the image in the image display area 310, a mark may be placed on the corresponding plot of the particle on the scattergram, and when the operator manipulates the plot of the particle on the scattergram, a mark may be placed on the corresponding image in the image display area 310.

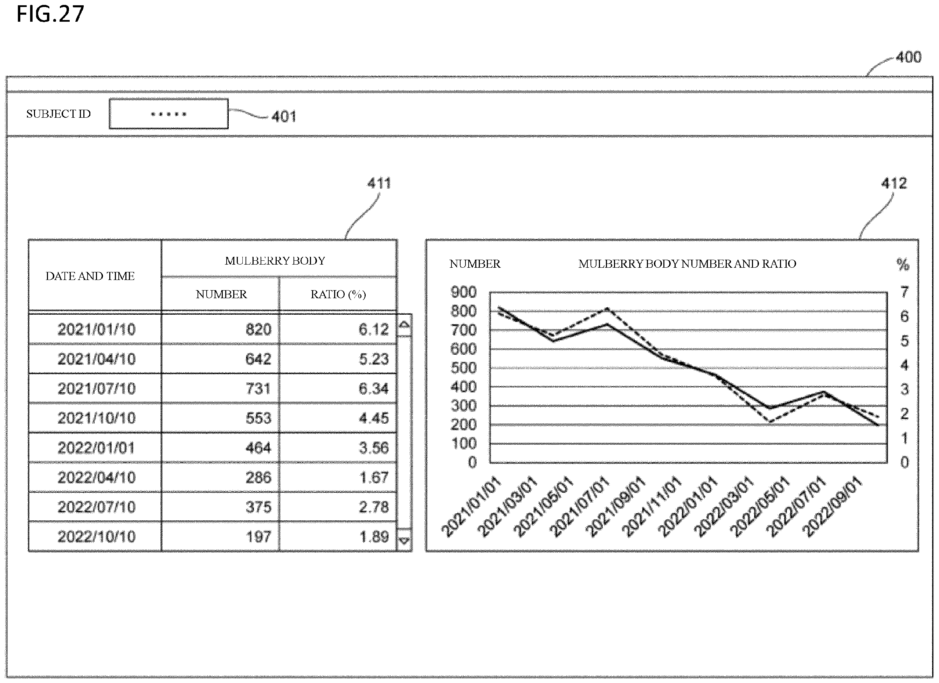

FIG. 27 is a diagram showing a schematic configuration of a screen 400 that displays the history and changes in the count results of mulberry bodies for the same subject.

The screen 400 includes a subject ID display area 401 , a list display area 411, and a graph display area 412 .

When the operator inputs the subject ID and then inputs an instruction to display the screen 400, the processor 11 displays the screen 400 shown in FIG. 27 on the display unit 14. At this time, the processor 11 makes an inquiry to the host computer (see FIG. 1) based on the subject ID, and receives a counting result corresponding to the subject ID from the host computer. When there are multiple counting results corresponding to one subject ID, the processing unit 11 receives the multiple count results as a history of the counting results from the host computer. The processing unit 11 displays the list display area 411 and the graph display area 412 based on the received counting results.

Each row of the list display area 411 corresponds to one counting result. The list display area 411 displays a number of particles determined to be mulberry bodies and a ratio of the number of particles determined to be mulberry bodies to a number of particles within the range R3. The graph display area 412 displays a graph based on the data in the list display area 411 . The graph in the graph display area 412 is information based on changes in the counting results.

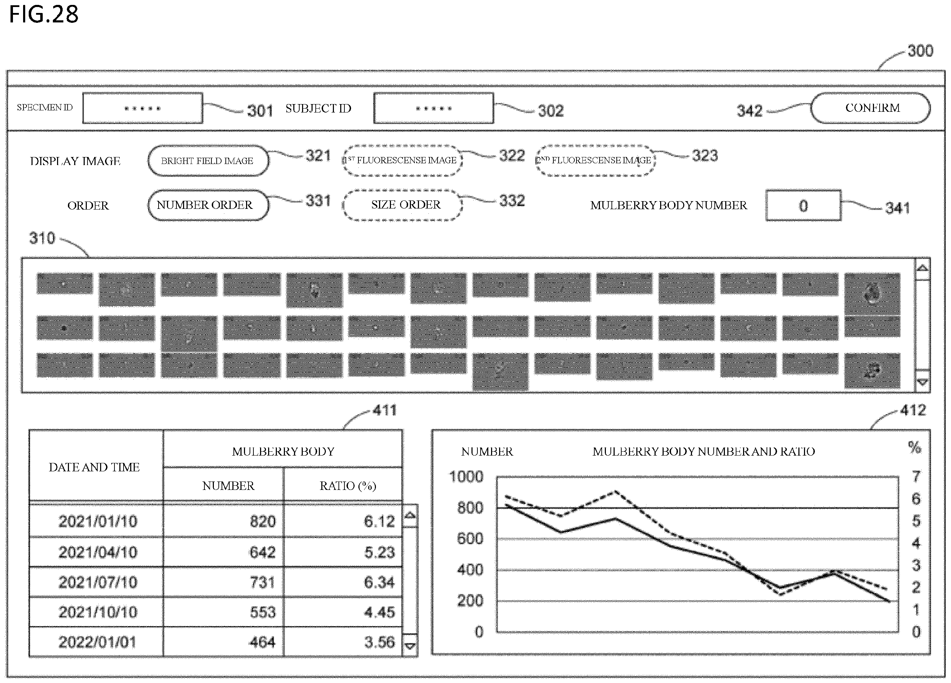

Although the list display area 411 and the graph display area 412 shown in FIG. 27 are displayed on the screen 400 separate from the screen 300, they may be displayed together with the screen 300 as shown in FIG. 28.

### <Effects of the particle detection method, particles detection apparatus, and program according to the embodiment>

In step S3 of FIG. 3, the processor 11 controls the light source 121 to irradiate the urine sample containing a plurality of types of particles with excitation light that causes autofluorescence from mulberry bodies. In addition, in step S3, the processor 11 disperses fluorescence generated from the urine sample irradiated with excitation light into fluorescence of a wavelength band including wavelength *λ* 21 (first wavelength band) and a wavelength band including wavelength *λ* 22 (second wavelength band), which are included in the wavelength band of the autofluorescence of mulberry bodies, and obtains fluorescence images for each particle. In step S4, the processor 11 extracts particles included in a range R1 corresponding to mulberry bodies based on reference information based on the fluorescence in the wavelength band including wavelength *λ* 21 (first wavelength band) and the fluorescence in the wavelength band including wavelength *λ* 22 (second wavelength band) obtained from the fluorescence images. The reference information is, for example, a maximum value, an average value, a total value, a median value, etc. of luminance of pixels identified as an area of a particle in the first and second fluorescent images.

According to this process, particles whose reference information based on the fluorescence of wavelength *λ* 21 (fluorescence in the first wavelength band) and the fluorescence of *λ* 22 (fluorescence in the second wavelength band) falling within the range R1 corresponding to mulberry bodies are extracted, thereby making it possible to more accurately distinguish mulberry bodies from other particles. Furthermore, this process eliminates the need for a step of staining particles in a urine specimen, making it possible to easily distinguish mulberry bodies.

The wavelength *λ* 11 of the excitation light is 350 nm or more and 550 nm or less.

This configuration allows autofluorescence to be generated from mulberry bodies.

The wavelength *λ* 11 of the excitation light is or is near 405 nm, the wavelength band including the wavelength *λ* 21 (first wavelength band) is 435 nm or more and 505 nm or less, and the wavelength band including the wavelength *λ* 22 (second wavelength band) is 505 nm or more and 560 nm or less.

According to this configuration, as shown in FIG. 4, mulberry bodies can be effectively discriminated from other particles.

The reference information based on the fluorescence in a wavelength band including wavelength *λ* 21 (first wavelength band) and the fluorescence in a wavelength band including wavelength 2. 22 (second wavelength band) obtained from the fluorescence images includes a first reference value related to an intensity of the fluorescence in the wavelength band including wavelength 2. 21 (first wavelength band) arising from a particle and a second reference value related to an intensity of the fluorescence in the wavelength band including wavelength *λ* 22 (second wavelength band) arising from the same particle. The first reference value is, for example, a maximum value, an average value, a total value, a median value, etc. of luminance of pixels identified as being in a region of a particle in the first fluorescent image. The second reference value is, for example, a maximum value, an average value, a total value, a median value, etc. of luminance of pixels identified as being in a region of a particle in the second fluorescent image.

According to this configuration, the range R1 corresponding to the mulberry bodies can be smoothly set.

The fluorescence images acquired by the imaging unit 20 includes a first fluorescence image capturing fluorescence in the wavelength band (first wavelength band) including wavelength 2. 21 generated from a particle, and a second fluorescence image capturing fluorescence in the wavelength band (second wavelength band) including wavelength *λ* 22 generated from the same particle.

According to this configuration, by separately imaging the fluorescence of wavelength *λ* 21 (fluorescence in the first wavelength band) and the fluorescence of wavelength *λ* 22 (fluorescence in the second wavelength band), it is possible to smoothly acquire the first reference value related to the intensity of the fluorescence of wavelength *λ* 21 (fluorescence in the first wavelength band) and the second reference value related to the intensity of the fluorescence of wavelength *λ* 22 (fluorescence in the second wavelength band).

The first reference value is, for example, a value related to luminance of a region of a particle in the first fluorescent image, and the second reference value is, for example, a value related to luminance of a region of the same particle in the second fluorescent image. The value related to luminance is, for example, a maximum value, an average value, a total value, a median value, etc. of luminance of pixels identified as an area of particle in the fluorescent image.

This configuration makes it easier to distinguish mulberry bodies from other particles.

The first reference value is a maximum luminance of a region of a particle in the first fluorescent image, and the second reference value is a maximum luminance of a region of the same particle in the second fluorescent image.

According to this configuration, as shown in FIG. 4, it becomes easier to discriminate mulberry bodies from other particles with high accuracy.

The step of extracting particles includes a first extraction step (a step) of extracting particles whose first and second reference values are included in the range R1 corresponding to mulberry bodies.

According to this configuration, particles included in the range R1 corresponding to mulberry bodies can be smoothly extracted.

As shown in Figure 4, when multiple particles are plotted on a scattergram 210 (coordinate system) having two axes, the vertical axis (coordinate axis corresponding to the first reference value) and the horizontal axis (coordinate axis corresponding to the second reference value), the range R1 corresponding to mulberry bodies is narrow near the origin of the scattergram 210 (coordinate system) and widens as the values on the vertical and horizontal axes (two axes) increase, and is a range sandwiched between a range R11 (first range) corresponding to a first type of particle other than mulberry bodies and a range R12 (second range) corresponding to a second type of particle other than mulberry bodies.

According to this configuration, in the first extraction process, a first type of particles distributed in the range R11 (e.g., squamous epithelial cells and tubular epithelial cells as exemplified in the upper part of Figure 6) and a second type of particles distributed in range R12 (e.g., salts and bacteria as exemplified in the lower part of Figure 6) can be excluded.

For example, as shown in FIG. 4, when multiple particles are plotted on a scattergram 210 (coordinate system) having two axes, a vertical axis (coordinate axis) corresponding to the first reference value and a horizontal axis (coordinate axis) corresponding to the second reference value, the range R1 corresponding to mulberry bodies is a range between a straight line R1a (first straight line) that has a first angle with respect to one of the vertical and horizontal axes (two axes) and passes through the origin of the scattergram 210 (coordinate system), and a straight line R1b (second straight line) that has a second angle with respect to the one axis that is different from the first angle and passes through the origin.

According to this configuration, mulberry bodies can be more accurately distinguished while excluding squamous epithelial cells, tubular epithelial cells, salts, bacteria, and the like.

In step S3 of FIG. 3, the processor 11 images the urine sample and obtains bright-field images of each particle. In the first extraction process of step S4, the processor 11 acquires the first reference value from the first fluorescent image, acquires the second reference value from the second fluorescent image, and extracts particles whose first reference value and second reference value are included in the range R1 corresponding to mulberry bodies. In the second extraction process of step S5, the processor 11 extracts particles whose other reference information obtained from the bright-field image are included in the range R2 (second range) corresponding to mulberry bodies.

This process allows for more accurate differentiation of mulberry bodies using bright-field images.

Other reference information described above includes a value of particle size and a value for particle contrast.

According to this configuration, particles in a urine specimen other than mulberry bodies (squamous epithelial cells distributed in ranges R21 and R22, urothelial cells distributed in range R23, salts and bacteria distributed in range R25) can be excluded based on values related to the size of the particles. The value relating to the contrast of particles makes it possible to exclude particles in the urine specimen other than mulberry bodies (squamous epithelial cells distributed in ranges R21 and R22, urothelial cells distributed in range R23, tubular epithelial cells, white blood cells, and red blood cells distributed in range R24).

In step S3 of FIG. 3, the processor 11 images the urine sample and obtains bright-field images of each particle. In the first extraction process of step S4, the processor 11 acquires the first reference value from the first fluorescent image, acquires the second reference value from the second fluorescent image, and extracts particles whose first reference value and second reference value are included in the range R1 corresponding to mulberry bodies. In the third extraction process of step S6, the processor 11 extracts particles whose value related to the aspect ratio of the particle based on the bright field image and first reference value are included in the range R3 (third range) corresponding to mulberry bodies.

According to this process, particles in a urine specimen other than mulberry bodies (particles such as casts distributed in range R32) can be excluded based on the value related to the aspect ratio of the particle. The first reference value (a value relating to luminance of a region of a particle in the first fluorescent image) in the third extraction step makes it possible to exclude particles in the urine specimen other than mulberry bodies (tubular epithelial cells, salts, fat globules, crystals, and bacteria distributed in range R31).

In step S3 of FIG. 3, the processor 11 images a urine sample and obtains bright-field images of each particle. In the first extraction process of step S4, the processor 11 acquires the first reference value from the first fluorescent image, acquires the second reference value from the second fluorescent image, and extracts particles whose first reference value and second reference value are included in the range R1 corresponding to mulberry bodies. In the second extraction process of step S5, the processor 11 extracts particles whose values related to size of particles and values related to contrast of particles based on the bright field image are included in the range R2 (second range) corresponding to mulberry bodies. In the third extraction process of step S6, the processor 11 extracts particles whose values related to aspect ratio of the particles based on the bright field image and the first reference value are included in the range R3 (third range) corresponding to mulberry bodies.

This process allows for more accurate discrimination of mulberry bodies.

The range R1 corresponding to mulberry bodies does not overlap with the ranges R11 and R12 corresponding to epithelial cells, salts, and bacteria.

According to this configuration, particles that generate autofluorescence in the same manner as mulberry bodies can be excluded, making it possible to more accurately distinguish mulberry bodies.

In step S7 of FIG. 3, the processor 11 displays images of particles extracted in the first to third extraction steps (steps of extracting particles) on the screen 300 shown in Figs. 23, 24, 26, and 28.

According to this process, an operator can smoothly identify particles that are highly likely to be mulberry bodies by referring to the images of the particles extracted in the first to third extraction steps.

In step S3 of Figure 3, the processor 11 captures images of multiple particles in the urine sample to obtain bright field images, and in step S7, displays the bright field images of the extracted particles in the image display area 310 shown in Figures 23, 24, 26, and 28.

Mulberry bodies are easy to spot in bright field images. Therefore, this process allows the operator to smoothly identify particles that are likely to be mulberry bodies by referring to the bright field image.

In step S7 in FIG. 3, the processor 11 displays a list of images of the extracted particles in the image display area 310 shown in Figs. 23, 24, 26, and 28.

According to this process, images of particles are displayed in a list, so that the operator can easily check the images of multiple particles in order.

In step S7 in FIG. 3, the processor 11 displays the extracted images of the particles in a list in the order of size of the particle images in the image display area 310, as shown in Figs. 24 and 26.

When the particles are rearranged in order of size, mulberry bodies are more likely to be displayed separately from other particles in the image display area 310. This allows the operator to more easily identify mulberry bodies.

In step S7 of Figure 3, the processor 11 accepts an instruction input to arrange the images of the extracted particles in order of the size of the particles via the size order button 332 shown in Figures 23, 24, 26, and 28, and rearranges the images of the particles displayed in a list in accordance with the received instruction input.

This process allows the operator to smoothly rearrange the displayed list of particles.

In step S7 of Figure 3, the processor 11 accepts an instruction input to identify any one of the images of the listed particles, and enlarges and displays the image of the particle identified by the instruction input, as shown in the enlarged image area 311 in Figure 24.

This process allows the operator to easily see the particles in detail, and therefore allows the operator to more accurately confirm whether the target particle is a mulberry body.

In step S7 of Figure 3, the processing unit 11 accepts an instruction input via the check box 311a in Figure 24 to identify an image of a mulberry body from the images of particles displayed in a list, and displays the counting result of counting the images of particles identified by the instruction input in the counting result display area 341, as shown in Figures 24 and 26.

According to this process, the number of particles that the operator identifies as mulberry bodies can be used as information that is useful for pathological diagnosis, such as determining whether or not a subject is suffering from Fabry disease.

The processor 11 stores the history of counting results for the same subject, as shown in the list display area 411 in Figs. 27 and 28.

By displaying such a history of counting results, the operator can appropriately check changes in a state of mulberry bodies in the subject.

As shown in the graph display area 412 in FIGS. 27 and 28, the processor 11 displays information based on changes in the counting results based on the history of the counting results.

This process allows an operator to smoothly ascertain changes in mulberry body count in a subject of interest. Therefore, it is possible to more accurately perform pathological diagnoses, such as determining whether or not a subject is suffering from Fabry disease and determining effects of medication on the subject.

As shown on a screen 300 in FIG. 28, the processor 11 displays a list of images of particles in the image display area 310, as well as information based on changes in the count results shown in the graph display area 412.

This process allows an operator to simultaneously view a list of images and changes in counting results. For example, it is possible to confirm whether medication given to a subject was effective.

In step S7 of Figure 3, the processor 11 accepts an instruction input via the check box 311a of Figure 24 to identify an image of a mulberry body from images of particles displayed in a list, and associates a flag with the image of the particle identified by the instruction input and stores it.

According to this process, images of particles that the operator has determined to be mulberry bodies can then be displayed together with the markings. This allows, for example, a doctor to smoothly check the results of the operator's determination at a later date. Therefore, the diagnosis can be performed more accurately.

As shown in FIG. 2, the particle detection apparatus 1 includes a light source 121 that irradiates a urine sample containing multiple types of particles with excitation light that causes autofluorescence from mulberry bodies, an imaging unit 20 that disperses fluorescence generated from the urine sample irradiated with the excitation light into fluorescence in a wavelength band including wavelength *λ* 21 (first wavelength band) and a wavelength band including wavelength *λ* 22 (second wavelength band) that are included in wavelength band of the autofluorescence, and acquires a fluorescence image for each particle, and a processor 11 that extracts particles whose reference information based on the fluorescence in the wavelength band including wavelength 2. 21 (first wavelength band) and the fluorescence in the wavelength band including wavelength 2. 22 (second wavelength band) obtained from the fluorescence image are included in a range R1 corresponding to mulberry bodies .

According to this configuration, particles whose reference information based on the fluorescence of wavelength *λ* 21 (fluorescence in the first wavelength band) and the fluorescence of wavelength *λ* 22 (fluorescence in the second wavelength band) are included in range R1 corresponding to mulberry bodies are extracted, thereby making it possible to more accurately distinguish mulberry bodies from other particles. Furthermore, according to this configuration, since a step of staining particles in the urine sample is not required, differentiation of mulberry bodies can be easily performed.

The imaging unit 20 includes a dichroic mirror of an optical unit 142 that separates the fluorescence generated from a urine sample irradiated with excitation light into fluorescence in a wavelength band including wavelength *λ* 21 (first wavelength band) and fluorescence in a wavelength band including wavelength *λ* 22 (second wavelength band).

According to this configuration, the fluorescence generated from the urine sample can be easily separated into the fluorescence of wavelength *λ* 21 and the fluorescence of wavelength *λ* 22.

The imaging unit 20 includes a flow cell 110 through which a urine sample flows, and images the urine sample flowing through the flow cell 110.

According to this configuration, images of a large number of particles can be efficiently acquired.

The program 12a (see FIG. 1) is a program that causes a computer to execute a process for detecting particles in a urine sample that contains a plurality of types of particles. When program 12a is executed by the processor 11, particles whose reference information based on fluorescence in a wavelength band including wavelength *λ* 21 (first wavelength band) and fluorescence in a wavelength band including wavelength *λ* 22 (second wavelength band) are included in range R1 corresponding to mulberry bodies are extracted. The first wavelength band and the second wavelength band are included in a wavelength band of autofluorescence of mulberry bodies. The autofluorescence of mulberry bodies are generated from a urine sample irradiated with excitation light that causes the autofluorescence. The fluorescence in the wavelength band including wavelength *λ* 21 (first wavelength band) and the fluorescence in the wavelength band including wavelength *λ* 22 (second wavelength band) are obtained from fluorescence images of each particle.

According to this configuration, particles whose reference information based on the fluorescence of wavelength *λ* 21 (fluorescence in the first wavelength band) and the fluorescence of wavelength *λ* 22 (fluorescence in the second wavelength band) are included in range R1 corresponding to mulberry bodies are extracted, thereby making it possible to more accurately distinguish mulberry bodies from other particles. Furthermore, according to this configuration, since a step of staining particles in the urine sample is not required, differentiation of mulberry bodies can be easily performed.

### <Modification 1>

In the above embodiment, the maximum luminance value is used in the first extraction step, but this is not limiting, and an average luminance value may be used. In the following, the first to third extraction steps of this modified example will be explained using the urine specimen with the specimen ID "F095" shown in FIG. 16 as an example.

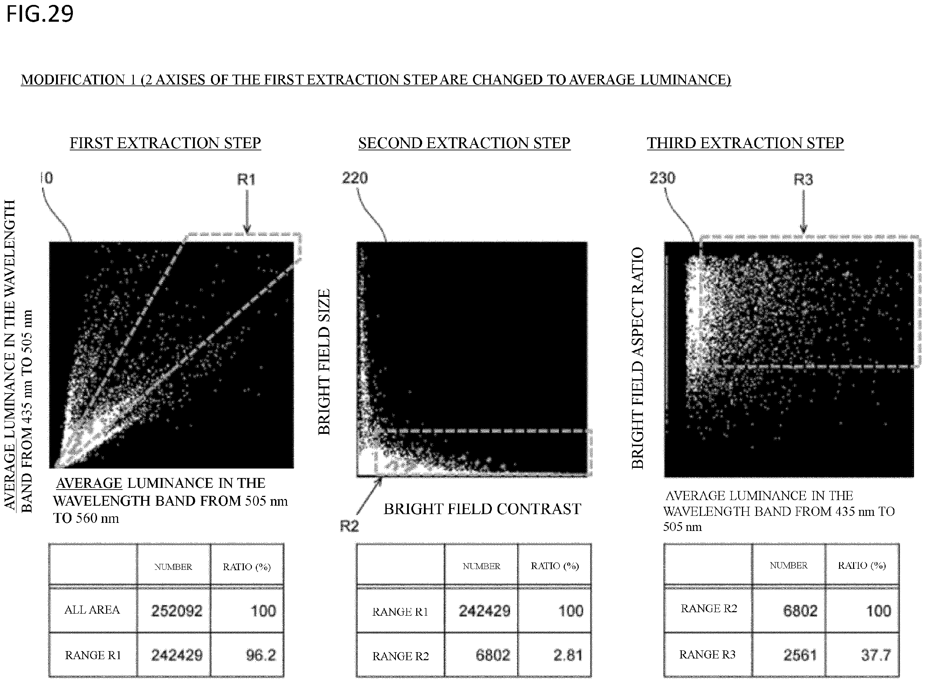

FIG. 29 is a diagram showing the first extraction step in which the average luminance is used according to this modification.

The scattergram 210 in FIG. 29 is generated based on all particles corresponding to images stored in the storage 12. In the scattergram 210, the vertical axis represents an average luminance of pixels identified as a particle in the first fluorescent image, and the horizontal axis represents an average luminance of pixels identified as a particle in the second fluorescent image. The range R1 in FIG. 29 is set to a range sandwiched between two straight lines extending obliquely from the origin, similar to the range R1 shown in FIG.

In the first extraction step in this case, 242,429 particles in the range R1 were extracted from the total of 252,092 particles. That is, of all the particles, 96.2% of the particles were extracted, and 3.8% of the particles were excluded. Then, based on the particles in the range R1 of FIG. 29, the second and third extraction steps are performed in the same manner as in the above embodiment. Thus, in the third extraction step, 2561 particles in the range R3 are extracted.

According to this modified example, a number of extractions using the range R1 in FIG. 29 (242,429 particles) is slightly greater than a number of extractions using the embodiment shown in FIG. 16 (236,893 particles), and a number of extractions using the range R3 in FIG. 29 (2,561 particles) is slightly greater than a number of extractions using the embodiment shown in FIG. 16 (2,061 particles); however, it can be said that particles were narrowed down to the same extent as in the above embodiment. Moreover, most of the gray plots indicating mulberry bodies are present within the ranges R1, R2, and R3. Therefore, in this modified example as well, the operator can smoothly and accurately select mulberry bodies by referring to the images of the extracted particles displayed on the screens 300 and 400.

### <Modification 2>

In the above embodiment, the average luminance value is used in the third extraction step, but this is not limiting, and the maximum luminance value may be used. In the following, the first to third extraction steps of this modified example will be explained using the urine specimen with the specimen ID "F095" shown in FIG. 16 as an example.

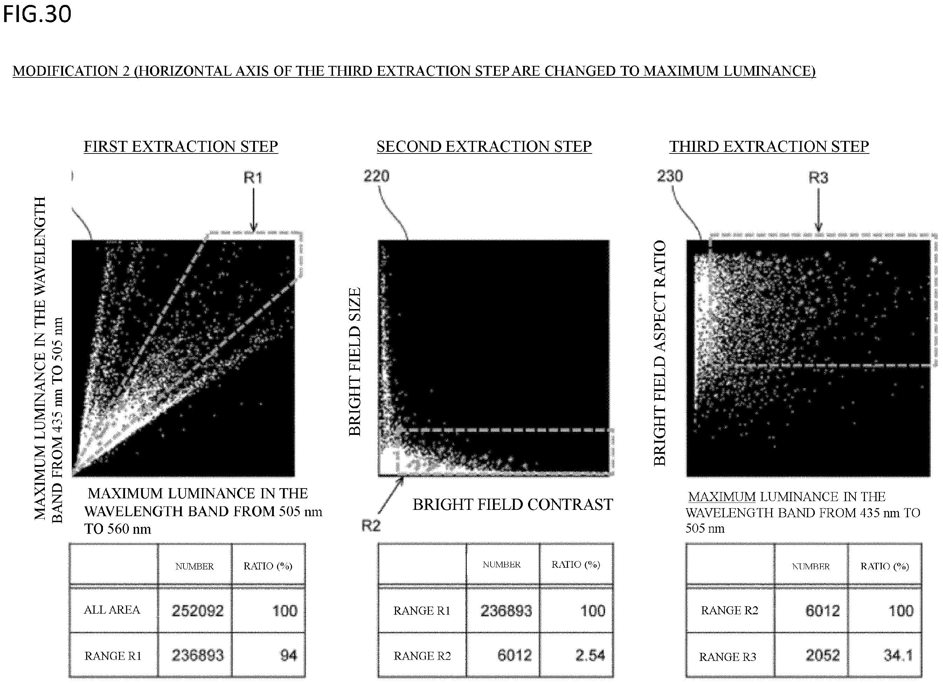

FIG. 30 is a diagram showing the third extraction step in which the maximum luminance is used according to this modification.

The scattergrams 210 and 220 in FIG. 30 are generated in the same manner as in Figs. 4 and 7. The scattergram 230 in FIG. 30 is generated based on particle in the range R2 of the scattergram 220 in FIG. 30. In the scattergram 230, the horizontal axis represents the maximum luminance of pixels identified as a particle in the first fluorescent image, and the vertical axis is the same as the vertical axis in FIG 4. The range R3 in FIG. 30 is set to a rectangular range, similar to the range R3 shown in FIG. 11.

In this case, in the third extraction step, 2052 particles in the range R3 were extracted from 6012 particles in the range R2. That is, of the particles in range R2, 34.1% of the particles were extracted and 65.9% of the particles were excluded.

According to this modified example, a number of extractions (2052 particles) in the range R3 in Figure 30 is almost the same as a number of extractions (2061 particles) in the above embodiment shown in Figure 16, so it can be said that particles were narrowed down to the same extent as in the above embodiment. Moreover, most of the gray plots indicating mulberry bodies are present within the ranges R1, R2, and R3. Therefore, in this modified example as well, the operator can smoothly and accurately select mulberry bodies by referring to images of the extracted particles displayed on the screens 300 and 400.

### <Modification 3>

In the above embodiment, the maximum luminance value is used in the first extraction step, and the average luminance value is used in the third extraction step. However, the present invention is not limited to this, and a total luminance values may be used in the first and third extraction steps. In the following, the first to third extraction steps of this modified example will be explained using the urine specimen with the specimen ID "F095" shown in FIG. 16 as an example.

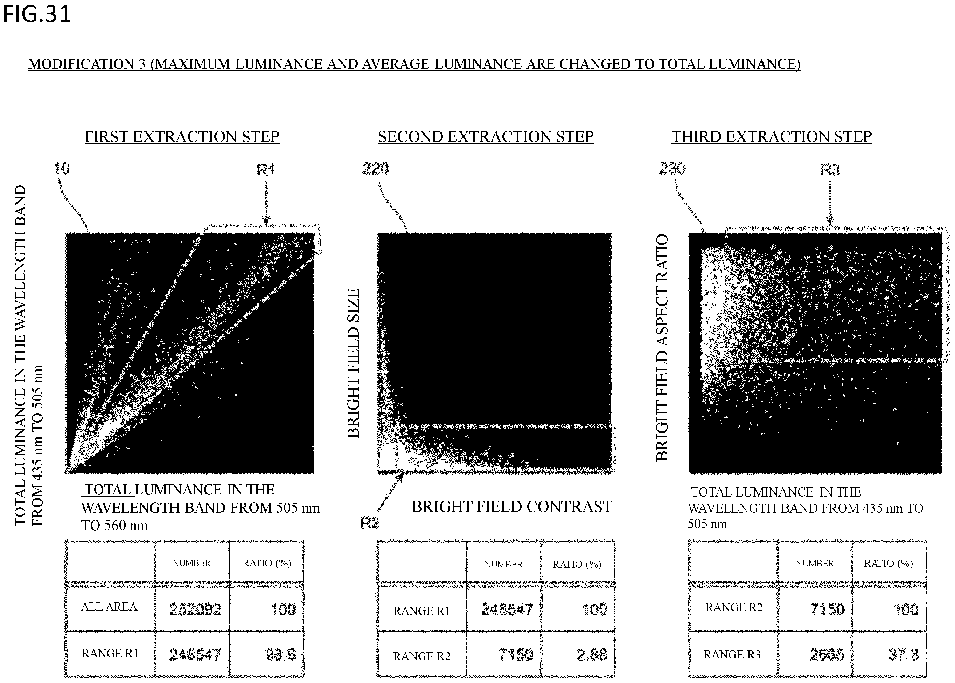

FIG. 31 is a diagram showing the first and third extraction steps in which the total luminance is used according to this modification.

The scattergram 210 in FIG. 31 is generated based on all particles corresponding to the images stored in the storage 12. In the scattergram 210, the vertical axis is the total luminance of pixels identified as a particle in the first fluorescent image, and the horizontal axis is the total luminance of pixels identified as a particle in the second fluorescent image. The range R1 in FIG. 31 is set to a range sandwiched between two straight lines extending obliquely, similar to the range R1 shown in FIG. 4.

In this case, in the first extraction step, 248,547 particles in the range R1 were extracted from the total of 252,092 particles. That is, of all the particles, 98.6% of the particles were extracted, and 1.4% of the particles were excluded. Then, based on the particles in the range R1 in FIG. 31, the second extraction step is performed in the same manner as in the above embodiment.

The scattergram 230 in FIG. 31 is generated based on the particle in the range R2 of the scattergram 220 in FIG. 31. In the scattergram 230, the horizontal axis represents a total luminance of pixels identified as a particle in the first fluorescent image, and the vertical axis is the same as the vertical axis in FIG. 4. The range R3 in FIG. 31 is set to a rectangular range, similar to the range R3 shown in FIG. 11.

In this case, in the third extraction step, 2665 particles in the range R3 were extracted from 7150 particles in the range R2. That is, of the particles in range R2, 37.3% of the particles were extracted and 62.7% of the particles were excluded.

According to this modified example, a number of extractions (2,665 particles) from the range R3 in Figure 31 is almost the same as a number of extractions (2,061 particles) in the above embodiment shown in Figure 16, so it can be said that particles were narrowed down to the same extent as in the above embodiment. Moreover, most of the gray plots indicating mulberry bodies are present within the ranges R1, R2, and R3. Therefore, in this modified example as well, the operator can smoothly and accurately select mulberry bodies by referring to images of the extracted particles displayed on the screens 300 and 400.

### <Modification 4>

In the above embodiment, both the bright-field size and the bright-field contrast are used in the second extraction step, but either one of the bright-field size and the bright-field contrast may be used. In the following, the second extraction step of this modified example will be described using the urine specimen with the specimen ID "F095" shown in Figure 16 as an example.

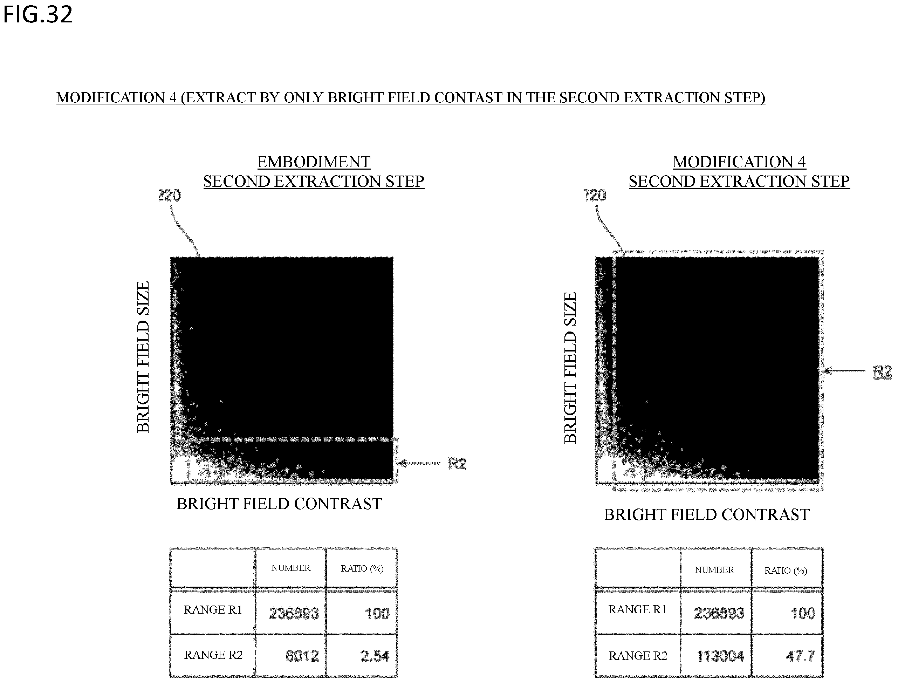

FIG. 32 shows a second extraction step in which only bright field contrast is used according to this modified example. The left side of FIG. 32 is a reference diagram illustrating the second extraction step of the embodiment, and the right side of FIG. 32 is a diagram illustrating the second extraction step of this modified example.

In the scattergram 220 of this modified example, the range R2 is set so as to include the entire bright field size (value on the vertical axis). That is, in this modified example, the second extraction step is performed using only bright field contrast.

In this case, in the second extraction step, 113,004 particles in the range R2 were extracted from the 236,893 particles in the range R1 in the first extraction step. That is, of the particles in the range R1, 47.7% of the particles were extracted and 52.3% of the particles were excluded.

According to this modified example, although a number of extractions in the range R2 (113,004 particles) was greater than a number of extractions in the embodiment (6,012 particles), it was possible to narrow down a number of particles in the second extraction step. Moreover, most of the gray plots indicating mulberry bodies are present within the range R2. Therefore, in this modified example as well, the operator can smoothly and accurately select mulberry bodies by referring to images of the extracted particles displayed on the screens 300 and 400.

### <Modification 5>

In the above embodiment, both the bright field aspect ratio and the average luminance value are used in the third extraction step, but either one of the bright field aspect ratio and the average luminance value may be used. In the following, the third extraction step of this modified example will be described using the urine specimen with the specimen ID "F095" shown in Figure 16 as an example.

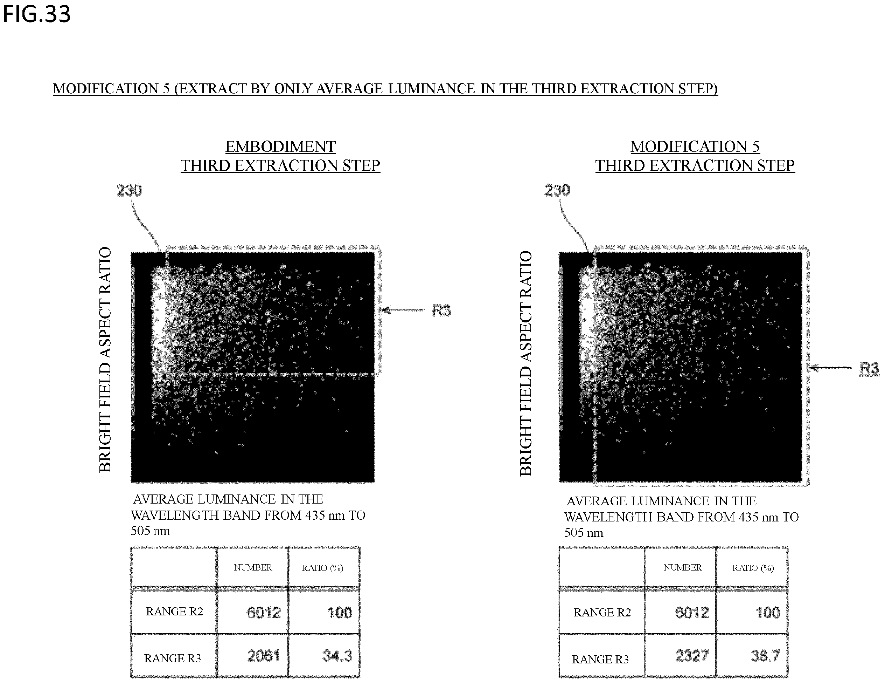

FIG. 33 is a diagram showing the third extraction step in which only the average luminance is used according to this modification. The left side of FIG. 33 is a reference diagram illustrating the third extraction step of the embodiment, and the right side of FIG. 33 is a diagram illustrating the third extraction step of this modified example.

In the scattergram 230 of this modified example, the range R3 is set so as to include all bright field aspect ratios (values on the vertical axis). That is, in this modified example, the third extraction step is performed using only the average luminance.

In this case, in the third extraction step, 2327 particles were extracted in the range R3 from the 6012 particles extracted in the range R2 in the second extraction step. That is, of the particles in the range R2, 38.7% of the particles were extracted and 61.3% of the particles were excluded.

According to this modified example, a number of extractions by the range R3 (2327 particles) is almost the same as a number of extractions (2061 particles) in the embodiment, so it can be said that particles were narrowed down to the same extent as in the above embodiment. Moreover, most of the gray plots indicating mulberry bodies are present within the range R3. Therefore, in this modified example as well, the operator can smoothly and accurately select mulberry bodies by referring to images of the extracted particles displayed on the screens 300 and 400.

### <Modification 6>

In the above-mentioned modified example 2, both the bright field aspect ratio and the maximum luminance value are used in the third extraction step, but either one of the bright field aspect ratio and the maximum luminance value may be used. In the following, the third extraction step of this modified example will be described using the urine specimen with the specimen ID "F095" shown in Figure 16 as an example.

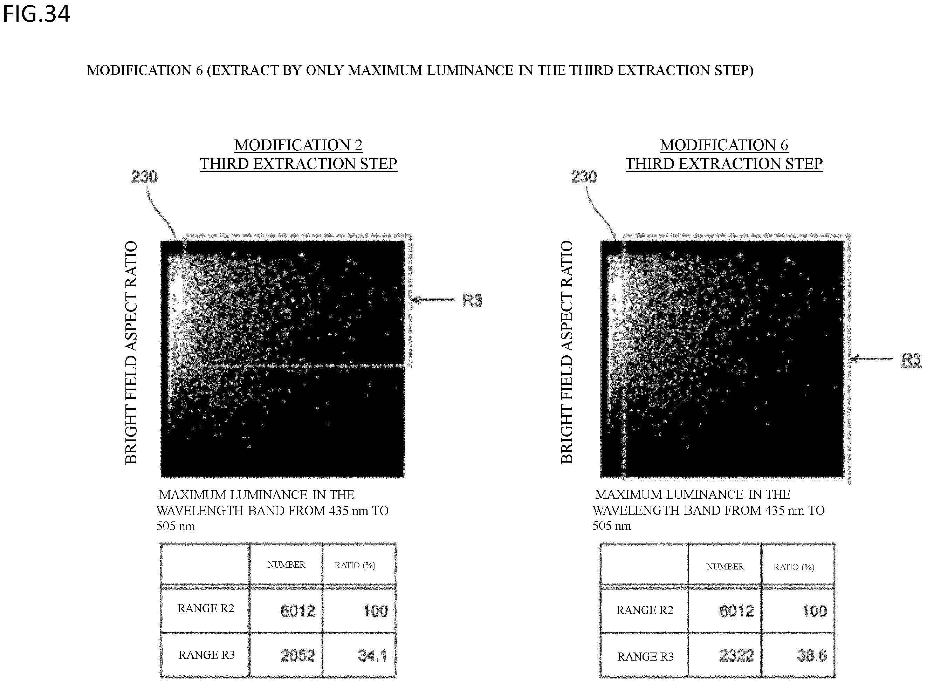

FIG. 34 is a diagram showing the third extraction step in which only the maximum luminance is used according to this modification. The left side of FIG. 34 is a reference diagram illustrating the third extraction step of the second modified example, and the right side of FIG. 34 is a diagram illustrating the third extraction step of this modified example.

In the scattergram 230 of this modified example, the range R3 is set so as to include all bright field aspect ratios (values on the vertical axis). That is, in this modified example, the third extraction step is performed using only the maximum luminance.

In this case, in the third extraction step, 2322 particles were extracted in the range R3 from the 6012 particles extracted in the range R2 in the second extraction step. That is, of the particles in the range R2, 38.6% of the particles were extracted and 61.4% of the particles were excluded.

According to this modified example, a number of extractions using range the R3 (2,322 particles) is almost the same as a number of extractions using modified example 2 (2,052 particles), so it can be said that particles were narrowed down to the same extent as in modified example 2. Moreover, most of the gray plots indicating mulberry bodies are present within the range R3. Therefore, in this modified example as well, the operator can smoothly and accurately select mulberry bodies by referring to images of the extracted particles displayed on the screens 300 and 400.

### <Modification 7>

In the above embodiment, the wavelength *λ* 11 of the excitation light emitted from the light source 121 is 405 nm, but this is not limited to this. The wavelength *λ* 11 may be any wavelength that generates autofluorescence from mulberry bodies when mulberry bodies are irradiated with excitation light.

In this modification, the wavelength *λ* 11 of the excitation light emitted from the light source 121 is set to 488 nm. In this case too, wavelength *λ* 11 indicates the wavelength at which an intensity of the light emitted from light source 121 is maximum, and the light of wavelength *λ* 11 emitted from light source 121 includes not only light of the wavelength at which the intensity of the light is maximum, but also light of wavelengths in the vicinity of the wavelength at which the intensity of the light is maximum.

When the wavelength *λ* 11 of the excitation light is 488 nm, the wavelength band of the autofluorescence generated from the mulberry bodies is 505 nm or more and 595 nm or less. Therefore, the imaging unit 20 of this modified example is configured to capture fluorescence with a wavelength *λ* 22 of 505 nm or more and 560 nm or less and fluorescence with a wavelength 2. 23 of 560 nm or more and 595 nm or less so that it can capture autofluorescence with a wavelength of 505 nm or more and 595 nm or less generated from mulberry bodies.

Specifically, the condenser lens 141 focuses the fluorescence of wavelengths *λ* 22 and *λ* 23 generated from the urine sample flowing through the flow path 111 of the flow cell 110, and the light of wavelength λ 12 that has been transmitted through the urine sample flowing through the flow path 111 of the flow cell 110. The three dichroic mirrors of the optical unit 142 reflect the fluorescence of wavelength *λ* 22, the fluorescence of wavelength *λ* 23, and the light of wavelength *λ* 12 at slightly different angles, thereby dispersing the light and separating it on the light receiving surface of the camera 144. The condenser lens 143 condenses the fluorescent light of wavelength λ 22, the fluorescent light of wavelength *λ* 23, and the light of wavelength *λ* 12.

Camera 144 captures the fluorescence of wavelength *λ* 22, the fluorescence of wavelength *λ* 23, and the light of wavelength *λ* 12, and outputs a first fluorescence image corresponding to the fluorescence of wavelength *λ* 22 (fluorescence in the first wavelength band), a second fluorescence image corresponding to the fluorescence of wavelength *λ* 23 (fluorescence in the second wavelength band), and a bright-field image corresponding to the light of wavelength *λ* 12 as imaging signals. As in the above embodiment, the processor 11 discriminates mulberry bodies contained in the urine specimen using the first fluorescent image, the second fluorescent image, and the bright-field image.

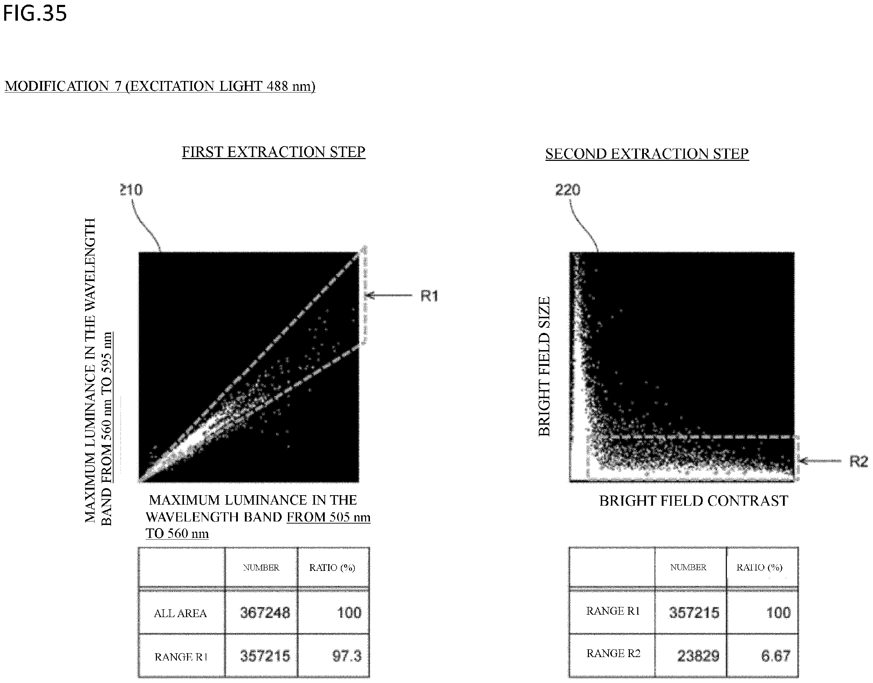

FIG. 35 is a diagram showing the first and second extraction steps according to this modified example.

The scattergram 210 of FIG. 35 is generated similarly to that of FIG. 4. The horizontal axis of the scattergram 210 in FIG. 35 represents the maximum luminance at wavelength *λ* 22 (a wavelength band of 505 nm or more and 560 nm or less), that is, the maximum luminance based on the first fluorescent image. The vertical axis of the scattergram 210 in FIG. 35 represents the maximum luminance at wavelength *λ* 23 (wavelength band of 560 nm or more and 595 nm or less), that is, the maximum luminance based on the second fluorescent image corresponding to wavelength 2. 23. The range R1 in FIG. 35 is set to a range sandwiched between two straight lines extending obliquely from the origin, similar to the range R1 in the embodiment shown in FIG. 4.

It should be noted that in the first extraction step of this modified example as well, the value relating to the intensity of the fluorescence of wavelength *λ* 22 generated from the particle is the maximum luminance, but is not limited to this, and may be, for example, an average value, a total value, or a median value of luminance of pixels identified as an area of a particle in the first fluorescent image. Similarly, the value relating to the intensity of the fluorescence of wavelength *λ* 23 from the particle is the maximum luminance, but is not limited to this, and may be, for example, an average, a total value, or a median of luminance of pixels identified as an particle in the second fluorescent image.

In the first extraction step in this case, 357215 particles in the range R1 were extracted from the total of 367248 particles. That is, of the total particles, 97.3% of the particles were extracted and 2.7% of the particles were excluded.

The scattergram 220 of FIG. 35 is generated in a similar manner to that of FIG. 7. In the second extraction step in this case, 23,829 particles in the range R2 were extracted from the 357,215 particles in the range R1. That is, of the total particles, 6.67% of the particles were extracted and 93.33% of the particles were excluded.

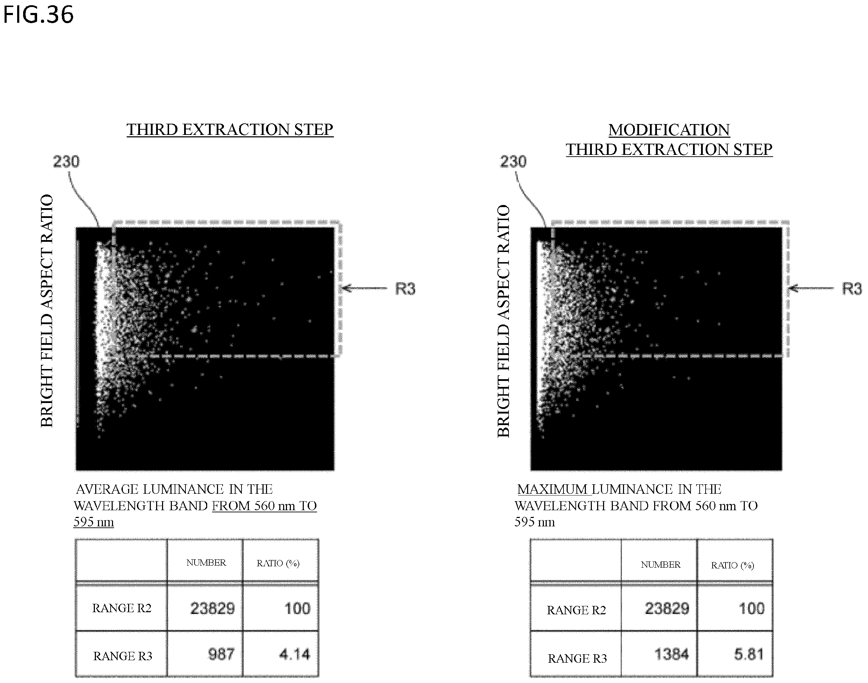

The left side of FIG. 36 is a diagram showing the third extraction step according to this modified example.

The scattergram 230 on the left side of FIG. 36 is generated similarly to FIG. 11. The horizontal axis of the scattergram 230 on the left side of FIG. 36 is the average brightness of pixels identified as areas of a particle in the second fluorescent image of wavelength *λ* 23 (wavelength band of 560 nm or more and 595 nm or less). The vertical axis of the scattergram 230 on the left side of FIG. 36 is the bright field aspect ratio, as in the embodiment shown in FIG. 11.

In this case, in the third extraction step, 987 particles in the range R3 were extracted from the 23,829 particles in the range R2. That is, of the particles in the range R 2, 4.14% of the particles were extracted and 95.86% of the particles were excluded.

According to this modified example, a number of extractions (987 particles) in the range R3 of the scattergram 230 on the left side of Figure 36 is slightly less than a number of extractions (2061 particles) in the above embodiment shown in Figure 16, so it can be said that particles were narrowed down more than in the above embodiment. Moreover, most of the gray plots indicating mulberry bodies are present within the ranges R1, R2, and R3. Therefore, in this modified example as well, the operator can smoothly and accurately select mulberry bodies by referring to images of the extracted particles displayed on the screens 300 and 400.

As shown on the right side of Figure 36, in the scattergram 230 on the left side of Figure 36, the horizontal axis may be set to the maximum luminance value of pixels identified as an area of a particle in the second fluorescent image.

In this case, 1,384 particles were extracted from the range R3 out of 23,829 particles extracted from the range R2. That is, of the particles in the range R2, 5.81% of the particles were extracted, and 94.19% of the particles were excluded. Since a number of extractions (1,384 particles) in the range R3 of scattergram 230 on the right side of Figure 36 is approximately the same as a number of extractions (987 particles) on the left side of Figure 36, it can be said that the modified example shown on the right side of Figure 36 was able to narrow down particles to the same extent as modified example 7 shown on the left side of Figure 36. Moreover, most of the gray plots indicating mulberry bodies are present within the range R3.

As described above, according to modification example 7, the wavelength *λ* 11 of the excitation light is in the vicinity of 488 nm, the wavelength band including the wavelength *λ* 22 (first wavelength band) is 505 nm or more and 560 nm or less, and the wavelength band including the wavelength *λ* 23 (second wavelength band) is 560 nm or more and 595 nm or less.

According to this configuration, as shown in FIG. 35, mulberry bodies can be discriminated from other particles.

### <Modification 8>

In the above embodiment, the first extraction step, the second extraction step, and the third extraction step are performed in this order as shown in FIG. 3. However, the order in which the first extraction step, the second extraction step, and the third extraction step are performed is not limited to this. For example, the second extraction step, the first extraction step, and the third extraction step may be performed in this order.

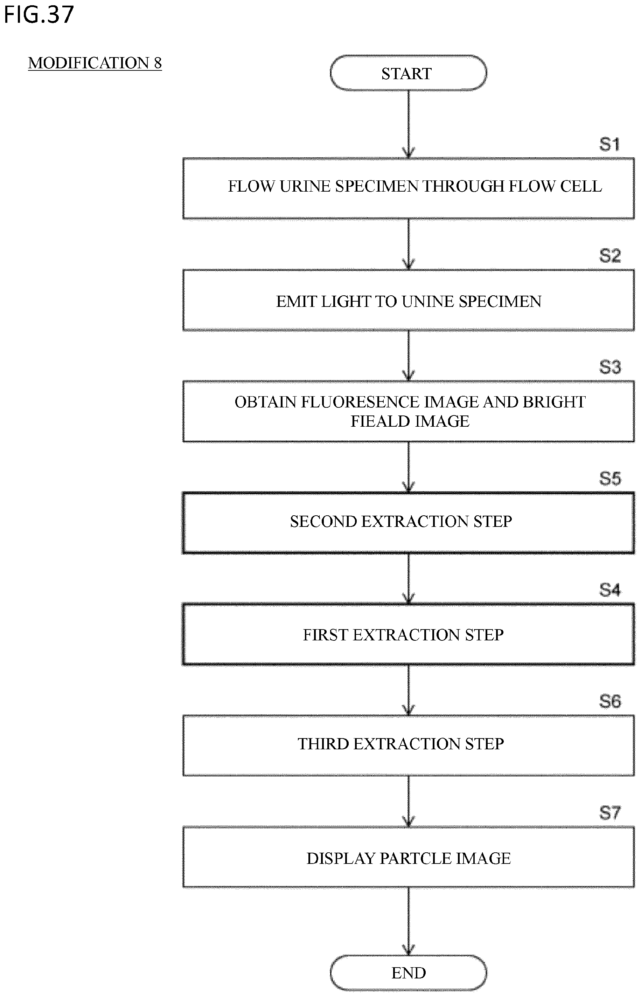

FIG. 37 is a flowchart showing the process of the particle detection method by the processor 11 according to this modified example.

In the process of FIG. 37, the order of the first extraction step in step S4 and the second extraction step in step S5 is reversed compared to the embodiment of FIG. 3.

In this case, in the second extraction process of step S5, the processor 11 extracts particles in the range R2 based on the bright-field image of all the particles acquired in step S3. Subsequently, in the first extraction process of step S4, the processor 11 extracts particles in the range R1 based on the first and second fluorescent images of the particles in the range R2 extracted in step S5. Then, in the third extraction process of step S6, the processor 11 extracts particles in the range R3 based on the first fluorescent image and the bright field image of the particles in the range R1 extracted in step S4. In this way, even if the execution order of the three extraction steps is changed, the final extracted particles are the same as those in the above embodiment.

However, as can be seen by referring to a number of particles in FIG. 16, a number of particles that can be removed in the second extraction step is several times greater than a number of particles that can be removed in the other extraction steps. In this case, as shown in FIG. 37, it is preferable that the second extraction step is performed prior to the first and third extraction steps. In this way, when the first of the three extraction processes is performed first, subsequent processing can be carried out with a reduced number of particles, thereby reducing an amount of calculations performed by the processor 11 in subsequent processes.

### <Modification 9>

In the above embodiment, of the three extraction steps, only the first extraction step and the second extraction step may be executed, only the first extraction step and the third extraction step may be executed, or only the first extraction step may be executed.

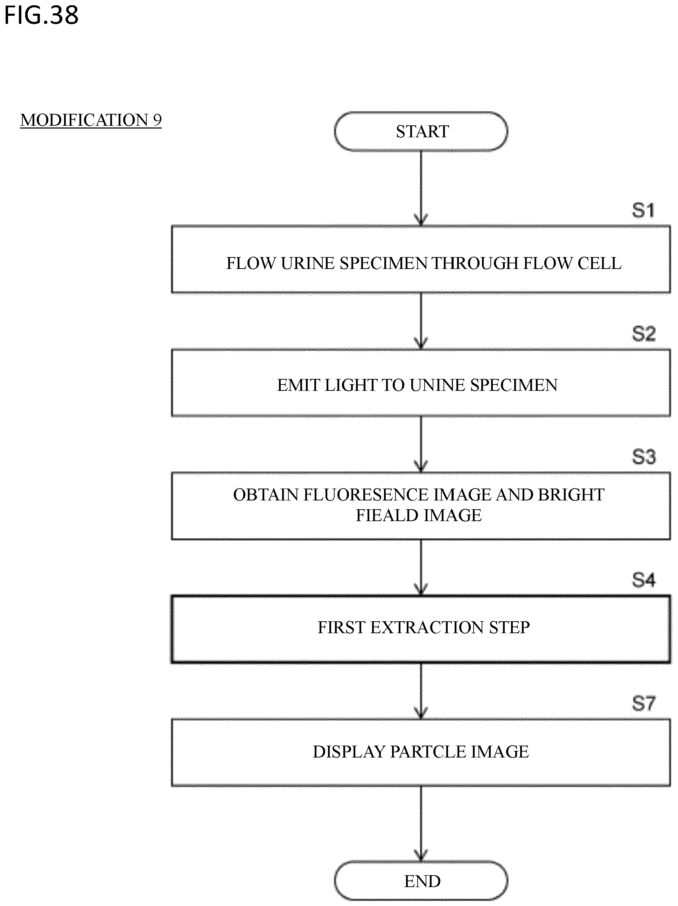

FIG. 38 is a flowchart showing the process of the particle detection method by the processor 11 in the case where only the first extraction step is executed according to this modified example.

In the process of FIG. 38, the second extraction step in step S5 and the third extraction step in step S6 are omitted compared to the embodiment of FIG. 3. In this case, in the first extraction process of step S4, the processor 11 extracts particles in the range R1 based on the first and second fluorescent images of all the particles acquired in step S3. Then, in step S7, the processor 11 displays on the screens 300, 400 images of the particles extracted in the first extraction process in

### step S4.

In this way, when only the first extraction step is performed as the extraction step, the number of particles displayed on the screens 300 and 400 becomes larger than when the first to third extraction steps are performed. However, even when only the first extraction process is performed, particles distributed in the range R1 corresponding to mulberry bodies are extracted, and particles distributed in the ranges R11 and R12 corresponding to particles other than mulberry bodies are excluded. Therefore, in this case as well, a number of particles displayed on the screens 300 and 400 will be smaller than in the case where the first to third extraction steps are not performed. Therefore, the operator can smoothly and accurately select mulberry bodies by referring to the screens 300 and 400.

### <Other modifications>

In the above embodiment and modified examples 1 to 6, 8, and 9, the wavelength band including the wavelength *λ* 21 is the wavelength band of 435 nm or more and 505 nm or less, but it is sufficient that it is included in the wavelength band of 435 nm or more and 505 nm or less. Furthermore, the wavelength band including the wavelength *λ* 22 is the wavelength band of 505 nm or more and 560 nm or less, but it is sufficient that it is included in the wavelength band of 505 nm or more and 560 nm or less. In this case as well, the mulberry bodies can be extracted by the first extraction step based on the first fluorescence image obtained from the fluorescence of wavelength *λ* 21 and the second fluorescence image obtained from the fluorescence of wavelength *λ* 22.

Similarly, in the seventh modification, the wavelength band including the wavelength *λ* 22 is the wavelength band of 505 nm or more and 560 nm or less, but it may be any wavelength band that is included in the wavelength band of 505 nm or more and 560 nm or less. Furthermore, the wavelength band including the wavelength *λ* 23 is the wavelength band of 560 nm or more and 595 nm or less, but it is sufficient that it is included in the wavelength band of 560 nm or more and 595 nm or less. In this case as well, the mulberry bodies can be extracted by the first extraction step based on the first fluorescence image obtained from the fluorescence of wavelength *λ* 22 and the second fluorescence image obtained from the fluorescence of wavelength *λ* 23.

In the above embodiment and modified examples 1 to 6, 8, and 9, the wavelength at the boundary between the wavelength band including the wavelength *λ* 21 and the wavelength band including the wavelength *λ* 22 was 505 nm, but this is not limited to this and it may be between the lower limit wavelength of the wavelength band including the wavelength *λ* 21 and the upper limit wavelength of the wavelength band including the wavelength *λ* 22. Similarly, in modification example 7, the wavelength at the boundary between the wavelength band including the wavelength *λ* 22 and the wavelength band including the wavelength *λ* 23 was 560 nm, but this is not limited to this and it may be between the lower wavelength of the wavelength band including the wavelength *λ* 22 and the upper wavelength of the wavelength band including the wavelength 2. 23. Even when the boundary wavelength is changed in this manner, the dichroic mirror of the optical unit 142 is configured to reflect the fluorescence of the two wavelength bands at angles slightly different from each other.

In the seventh modified example, the value related to the intensity of the fluorescent light having the wavelength *λ* 23 is used in the third extraction step. However, instead of this, a value related to the intensity of the fluorescent light having the wavelength *λ* 22 may be used.

In the above embodiment and modified examples 1 to 9, the imaging unit 20 is configured to acquire autofluorescence in two wavelength bands out of the autofluorescence in the specified wavelength bands generated by mulberry bodies, but the present invention is not limited to this and may be configured to acquire autofluorescence in three or more wavelength bands out of the autofluorescence in the specified wavelength bands generated by mulberry bodies. In this case, in the first extraction step, the processing unit 11 extracts particles contained in the range corresponding to mulberry bodies using reference information obtained from fluorescent images captured by capturing the fluorescence of three or more wavelength bands.

In the above embodiment and modified examples 1 to 9, the ranges R1, R2, and R3 are fixed ranges, but at least one of the ranges R1, R2, and R3 may be a variable range. For example, at least one of the ranges R1, R2, and R3 may be a range that changes depending on the distribution state of the particles in the scattergram.

In the above embodiment and modified examples 1 to 3 and 7, the range R1 is the range surrounded by two straight lines R1a and R1b (see FIG. 4). However, this is not limited to the above, and range R1 may be narrow near the origin of scattergram 210, widen as the values on the vertical and horizontal axes increase, and be sandwiched between ranges R11 and R12 corresponding to particles other than mulberry bodies, and the boundary line of range R1 may include vertical or horizontal straight lines or curves.

In the above embodiment and modified examples 1 to 9, information such as bright-field contrast, bright-field size, and bright-field aspect ratio is obtained from the bright-field image, but other information such as bright-field focus may also be obtained. The bright field focus is an example of a value related to the focus state of a particle based on a bright field image. Bright-field focus is, for example, a value (average gradient) calculated by normalizing luminance of pixels identified as a region of a particle in a bright-field image, and then dividing the sum of the luminance gradients between adjacent pixels by the number of pixels. The larger the bright field focus value, the more focused the particles are in the bright field image. Brightfield focus can be used to eliminate fuzzy, out-of-focus particles in the brightfield image.

In the above embodiment and modified examples 1 to 9, the values on the vertical and horizontal axes in the first extraction process are values relating to the intensity of the same type of fluorescence, but this is not limited thereto and the values may be values relating to the intensity of different types of fluorescence. For example, the vertical axis may represent the average luminance and the horizontal axis may represent the maximum luminance.

In the above embodiment and modified examples 1 to 9, the excitation light emitted from the light source 121 is laser light having a narrow wavelength range over which the intensity increases, but this is not limited thereto, and the excitation light may be light (e.g., LED light) having a wavelength range over which the intensity increases that is wider than that of laser light.

In the above embodiment and modified examples 1 to 6, 8, and 9, values related to the intensity of the fluorescence at wavelengths *λ* 21 and *λ* 22 generated from the particles are obtained from the first and second fluorescent images, respectively. However, this is not limiting, and both the fluorescence of wavelength *λ* 21 and the fluorescence of wavelength *λ* 22 may be guided in a separated state onto the same light receiving surface of camera 144, and camera 144 may capture these two fluorescent lights to generate a single fluorescent image. In this case, for example, the processor 11 obtains both a value relating to the intensity of the fluorescence at wavelength *λ* 21 and a value relating to the intensity of the fluorescence at wavelength *λ* 22 from one generated fluorescence image through image processing.

Similarly, in modification example 7, both the fluorescence of wavelength *λ* 22 and the fluorescence of wavelength *λ* 23 may be directed in a separated state onto the same light receiving surface of camera 144, and camera 144 may capture these two fluorescent lights to generate a single fluorescent image. In this case as well, for example, the processor 11 obtains both a value relating to the intensity of the fluorescence at wavelength *λ* 22 and a value relating to the intensity of the fluorescence at wavelength *λ* 23 from one generated fluorescence image through image processing.

In the above embodiment and modifications 1 to 9, as shown in FIG. 2, the imaging unit 20 includes the flow cell 110, and images of a plurality of particles in the urine sample flowing through the flow cell 110 are captured. However, the present invention is not limited to this, and the imaging unit 20 may be configured by a microscope. In this case, the microscope images a plurality of particles in the urine sample on the slide and generates at least a fluorescent image, preferably a first fluorescent image, a second fluorescent image and a bright field image, for each particle. The processor 11 uses the image acquired by the microscope to extract mulberry bodies in the same manner as in the above embodiment.

In the above embodiment and modified examples 1 to 9, the image of the particle is displayed in step S7, but the process of step S7 may be omitted. In this case, for example, the particles extracted by the first to third extraction steps may be subjected to image analysis by the processor 11 or another device to determine the possibility that each particle is a mulberry body.

The embodiments of the present invention can be modified in various ways as appropriate within the scope of the technical ideas defined in the claims.

### <Remarks>

The present disclosure includes following items 1-33.

Item 1: A particle detection method comprising:
irradiating a urine sample containing multiple types of particles with excitation light to cause autofluorescence from mulberry bodies;
dispersing fluorescence generated from the urine sample irradiated with the excitation light into fluorescence in a first wavelength band and fluorescence in a second wavelength band, and acquiring fluorescence images for each of the particles, the first wavelength band and the second wavelength band being included in a wavelength band of the autofluorescence; and
extracting a particle whose reference information based on the fluorescence in the first wavelength band and the fluorescence in the second wavelength band obtained from the corresponding fluorescence image is included in a range corresponding to the mulberry bodies.

Item 2: The particle detection method of item 1, wherein wavelength of the excitation light is 350 nm to 550 nm.

Item 3: The particle detection method according to item 2, wherein wavelength of the excitation light is near 405 nm, the first wavelength band is included in wavelength band of 435 nm to 505 nm, and the second wavelength band is included in wavelength band of 505 nm to 560 nm.

Item 4: The particle detection method according to item 2, wherein wavelength of the excitation light is near 488 nm, the first wavelength band is included in wavelength band of 505 nm to 560 nm, and the second wavelength band is included in wavelength band of 560 nm to 595 nm.

Item 5: The particle detection method according to item 1, wherein the reference information includes a first reference value regarding intensity of the fluorescence in the first wavelength band generated from the particle and a second reference value regarding intensity of the fluorescence in the second wavelength band generated from the same particle.

Item 6: The particle detection method according to item 5, wherein the fluorescence images include a first fluorescence image capturing the fluorescence in the first wavelength band generated from the particle and a second fluorescence image capturing the fluorescence in the second wavelength band generated from the same particle.

Item 7: The particle detection method according to item 6, wherein the first reference value is a value regarding brightness of a region of the particle in the first fluorescence image, and the second reference value is a value regarding brightness of a region of the same particle in the second fluorescence image.

Item 8: The particle detection method according to item 7, wherein the first reference value is the maximum brightness of the region of the particle in the first fluorescence image, and the second reference value is the maximum brightness of the region of the same particle in the second fluorescence image.

Item 9: The particle detection method according to item 5, wherein the extracting the particle includes extracting a particle whose first reference value and second reference value are included in the range corresponding to the mulberry bodies.

Item 10: The particle detection method according to item 9, wherein the range corresponding to the mulberry bodies is a range that is narrow near an origin of a coordinate system having an coordinate axis corresponding to the first reference value and an coordinate axis corresponding to the second reference value as two axes, and widens as the values of the two axes increase, and is sandwiched between a first range corresponding to a first type of particles other than the mulberry bodies and a second range corresponding to a second type of particles other than the mulberry bodies, when plotting the multiple particles in the coordinate system.

Item 11: The particle detection method according to item 9, wherein the range corresponding to the mulberry bodies is a range between a first line passing through an origin with a first angle with respect to one of two axes in a coordinate system having an coordinate axis corresponding to the first reference value and an coordinate axis corresponding to the second reference value as the two axes and a second line passing through the origin with a second angle different from the first angle with respect to the one of the two axis, when plotting the multiple particles in the coordinate system.

Item 12: The particle detection method according to item 6, further comprising: capturing each of the particles in the urine sample, to obtain bright field images, wherein the extracting the particle includes: obtaining the first reference value from the first fluorescence image, obtaining the second reference value from the second fluorescence image, and extracting a particle whose first reference value and second reference value are included in the range corresponding to the mulberry bodies; and extracting a particle whose other reference information obtained from the bright field image is included in a second range corresponding to the mulberry bodies.

Item 13: The particle detection method according to item 12, wherein the other reference information includes at least one of a value regarding size of the particle and a value regarding contrast of the particle.

Item 14: The particle detection method according to item 6, further comprising: capturing each of the particles in the urine sample, to obtain bright field images, wherein the extracting the particle includes: obtaining the first reference value from the first fluorescence image, obtaining the second reference value from the second fluorescence image, and extracting a particle whose first reference value and second reference value are included in the range corresponding to the mulberry bodies; and extracting a particle whose at least one of a value regarding aspect ratio of the particle based on the bright field image and the first reference value is included in a third range corresponding to the mulberry bodies.

Item 15: The particle detection method according to item 6, further comprising: capturing each of the particles in the urine sample, to obtain bright field images, wherein the extracting the particle includes: a first extraction step of obtaining the first reference value from the first fluorescence image, obtaining the second reference value from the second fluorescence image, and extracting a particle whose first reference value and second reference value are included in the range corresponding to the mulberry bodies; a second extraction step of extracting a particle whose at least one of a value regarding size of the particle based on the bright field image and a value regarding contrast of the particle is included in a second range corresponding to the mulberry bodies; and a third extraction step of extracting a particle whose at least one of a value regarding aspect ratio of the particle based on the bright field image and the first reference value is included in a third range corresponding to the mulberry bodies.

Item 16: The particle detection method according to item 15, wherein the first extraction step, the second extraction step, and the third extraction step are such that extraction is executed with respect to a group of particles having been extracted through a previously-executed one of the extraction steps.

Item 17: The particle detection method according to item 15, wherein the second extraction step is executed prior to the first extraction step and the third extraction step.

Item 18: The particle detection method according to item 1, wherein the range corresponding to the mulberry bodies is a range that overlaps with neither a range corresponding to epithelial cells, a range corresponding to salts, nor a range corresponding to bacteria.

Item 19: The particle detection method according to item 1, further comprising: displaying an image of the particle extracted by the step of extracting the particle.

Item 20: The particle detection method according to item 19, further comprising: capturing the particles in the urine sample, to obtain bright field images, wherein the displaying the image of the particle includes displaying the bright field images of the extracted particles.

Item 21: The particle detection method according to item 19, wherein displaying the image of the particle includes displaying the images of the extracted particles in a list.

Item 22: The particle detection method according to item 21, wherein displaying the image of the particle includes displaying the image of the extracted particles in the list in order of sizes of the particles.

Item 23: The particle detection method according to item 21, wherein the displaying of the image of the particle includes receiving input of an instruction to arrange the images of the extracted particles in an order of sizes of the particles, and rearranging the listed images of the particles according to the received input of the instruction.

Item 24: The particle detection method according to citem 21, wherein the displaying of the image of the particle includes receiving input of an instruction to specify any one of the listed images of the particles, and displaying, in an enlarged manner, the image of the particle specified through the input of the instruction.

Item 25: The particle detection method according to item 21, wherein the displaying of the image of the particle includes receiving input of an instruction to specify images of the mulberry bodies among the listed images of the particles, and further displaying a counting result obtained by counting the images of the particles specified through the input of the instruction.

Item 26: The particle detection method according to item 25, further comprising storing histories of the counting results regarding a same subject.

Item 27: The particle detection method according to item 26, further comprising displaying information based on change in the counting results on the basis of the histories.

Item 28: The particle detection method according to item 27, further comprising displaying the information based on the change in the counting results together with the listed images of the particles.

Item 29: The particle detection method according to item 21, wherein the displaying of the image of the particle includes receiving input of an instruction to specify an image of any of the mulberry bodies among the listed images of the particles, and storing the image of the particle specified through the input of the instruction such that a flag is associated with the image.

Item 30: A particle detection apparatus, comprising:
a light source for irradiating a urine sample containing multiple types of particles with excitation light to cause autofluorescence from mulberry bodies;
an imaging unit for dispersing fluorescence generated from the urine sample irradiated with the excitation light into fluorescence in a first wavelength band and fluorescence in a second wavelength band, and for acquiring fluorescence images for each of the particles, the first wavelength band and the second wavelength band being included in a wavelength band of the autofluorescence; and
a processor for extracting a particle whose reference information based on the fluorescence in the first wavelength band and the fluorescence in the second wavelength band obtained from the corresponding fluorescence image is included in a range corresponding to the mulberry bodies.

Item 31: The particle detection apparatus according to item 30, wherein the imaging unit comprises a dichroic mirror for dispersing the fluorescence generated from the urine sample irradiated with the excitation light into the fluorescence in the first wavelength band and the fluorescence in the second wavelength band.

Item 32: The particle detection apparatus according to item 30, wherein the imaging unit comprises a flow cell through which the urine sample flows and captures the urine sample flowing through the flow cell.

Item 33: A program configured to cause a computer to execute a process of detecting particles in a urine sample containing multiple types of particles, the process comprising:
extracting a particle whose reference information based on fluorescence in a first wavelength band and fluorescence in a second wavelength band obtained from corresponding fluorescence image is included in a range corresponding to mulberry bodies, wherein the first wavelength band and the second wavelength band being included in a wavelength band of autofluorescence, the autofluorescence is caused from a urine sample to which excitation light to cause the autofluorescence from the mulberry bodies is irradiated.

## Claims

1. A particle detection method comprising:
irradiating a urine sample containing multiple types of particles with excitation light to cause autofluorescence from mulberry bodies;
dispersing fluorescence generated from the urine sample irradiated with the excitation light into fluorescence in a first wavelength band and fluorescence in a second wavelength band, and acquiring fluorescence images for each of the particles, the first wavelength band and the second wavelength band being included in a wavelength band of the autofluorescence; and
extracting a particle whose reference information based on the fluorescence in the first wavelength band and the fluorescence in the second wavelength band obtained from the corresponding fluorescence image is included in a range corresponding to the mulberry bodies.

2. The particle detection method according to claim 1, wherein
the reference information includes a first reference value regarding intensity of the fluorescence in the first wavelength band generated from the particle and a second reference value regarding intensity of the fluorescence in the second wavelength band generated from the same particle.

3. The particle detection method according to claim 2, wherein
the fluorescence images include a first fluorescence image capturing the fluorescence in the first wavelength band generated from the particle and a second fluorescence image capturing the fluorescence in the second wavelength band generated from the same particle.

4. The particle detection method according to claim 3, wherein
the first reference value is a value regarding brightness of a region of the particle in the first fluorescence image, and the second reference value is a value regarding brightness of a region of the same particle in the second fluorescence image.

5. The particle detection method according to claim 2, wherein
the extracting the particle includes extracting a particle whose first reference value and the second reference value are included in the range corresponding to the mulberry bodies.

6. The particle detection method according to claim 5, wherein
the range corresponding to the mulberry bodies is a range that is narrow near an origin of a coordinate system having an coordinate axis corresponding to the first reference value and an coordinate axis corresponding to the second reference value as two axes, and widens as the values of the two axes increase, and is sandwiched between a first range corresponding to a first type of particles other than the mulberry bodies and a second range corresponding to a second type of particles other than the mulberry bodies, when plotting the multiple particles in the coordinate system.

7. The particle detection method according to claim 5, wherein
the range corresponding to the mulberry bodies is a range between a first line passing through an origin with a first angle with respect to one of two axes in a coordinate system having an coordinate axis corresponding to the first reference value and an coordinate axis corresponding to the second reference value as the two axes and a second line passing through the origin with a second angle different from the first angle with respect to the one of the two axis, when plotting the multiple particles in the coordinate system.

8. The particle detection method according to claim 3, further comprising:
capturing each of the particles in the urine sample, to obtain bright field images, wherein
the extracting the particle includes:
obtaining the first reference value from the first fluorescence image, obtaining the second reference value from the second fluorescence image, and extracting a particle whose first reference value and second reference value are included in the range corresponding to the mulberry bodies; and
extracting a particle whose other reference information obtained from the bright field image is included in a second range corresponding to the mulberry bodies.

9. The particle detection method according to claim 8, wherein
the other reference information includes at least one of a value regarding size of the particle and a value regarding contrast of the particle.

10. The particle detection method according to claim 3, further comprising:
capturing each of the particles in the urine sample, to obtain bright field images, wherein
the extracting the particle includes:
obtaining the first reference value from the first fluorescence image, obtaining the second reference value from the second fluorescence image, and extracting a particle whose first reference value and second reference value are included in the range corresponding to the mulberry bodies; and
extracting a particle whose at least one of a value regarding aspect ratio of the particle based on the bright field image and the first reference value is included in a third range corresponding to the mulberry bodies.

11. The particle detection method according to claim 3, further comprising:
capturing each of the particles in the urine sample, to obtain bright field images, wherein
the extracting the particle includes:
a first extraction step of obtaining the first reference value from the first fluorescence image, obtaining the second reference value from the second fluorescence image, and extracting a particle whose first reference value and second reference value are included in the range corresponding to the mulberry bodies;
a second extraction step of extracting a particle whose at least one of a value regarding size of the particle based on the bright field image and a value regarding contrast of the particle is included in a second range corresponding to the mulberry bodies; and
a third extraction step of extracting a particle whose at least one of a value regarding aspect ratio of the particle based on the bright field image and the first reference value is included in a third range corresponding to the mulberry bodies.

12. A particle detection apparatus, comprising:
a light source for irradiating a urine sample containing multiple types of particles with excitation light to cause autofluorescence from mulberry bodies;
an imaging unit for dispersing fluorescence generated from the urine sample irradiated with the excitation light into fluorescence in a first wavelength band and fluorescence in a second wavelength band, and for acquiring fluorescence images for each of the particles, the first wavelength band and the second wavelength band being included in a wavelength band of the autofluorescence; and
a processor for extracting a particle whose reference information based on the fluorescence in the first wavelength band and the fluorescence in the second wavelength band obtained from the corresponding fluorescence image is included in a range corresponding to the mulberry bodies.

13. The particle detection apparatus according to claim 12, wherein
the imaging unit comprises a dichroic mirror for dispersing the fluorescence generated from the urine sample irradiated with the excitation light into the fluorescence in the first wavelength band and the fluorescence in the second wavelength band.

14. The particle detection apparatus according to claim 12, wherein
the imaging unit comprises a flow cell through which the urine sample flows and captures the urine sample flowing through the flow cell.

15. A program configured to cause a computer to execute a process of detecting particles in a urine sample containing multiple types of particles, the process comprising:
extracting a particle whose reference information based on fluorescence in a first wavelength band and fluorescence in a second wavelength band obtained from corresponding fluorescence image is included in a range corresponding to mulberry bodies, wherein the first wavelength band and the second wavelength band being included in a wavelength band of autofluorescence, the autofluorescence is caused from a urine sample to which excitation light to cause the autofluorescence from the mulberry bodies is irradiated.
